# EUROPEAN PATENT APPLICATION

(11) **EP 1 990 417 A1**
(43) Date of publication of application: **12.11.2008**
(21) Application number: 07009507.0
(22) Date of filing: 11.05.2007
(51) Int. Cl.: C12N 15/74, C12N 15/09

(54) **Archaeal plasmid vector system**

(71) Applicant: Universität Bayreuth, 95440 Bayreuth (DE)
(72) Inventor: Lipps, Georg, 95447 Bayreuth (DE); Berkner, Silvia, 95447 Bayreuth (DE)
(74) Representative: Graf von Stosch, Andreas

(57) **Abstract**

The present invention relates to an archaeal plasmid vector system comprising: I) one or more selectable marker gene(s) for selection in archaea, and II) a component selected from a) an archaeal origin of replication, b) a homology region to an archaeal chromosome, c) a gene coding for a molecule conferring the ability to integrate the vector into an archaeal chromosome, and d) the gene *orf904;* wherein the genes of the plasmid vector do not allow an assembly of a functional virus particle. Further, the present invention relates to a plasmid vector system comprising: I) the gene *orf904,* and II) one or more selectable marker gene(s); wherein the vector is non-integrative. Moreover, the present invention relates to host cells transformed with the vector system as well as to kits comprising the vector system or a host cell of the present invention. Furthermore, the present application also relates to a method for producing a protein of interest.

SEQUENCE LISTING

<110> Universität Bayreuth

<120> Archael Plasmid Vector System

<130> UB03P001EP

<140> EP 07009507.0
<141> 2007-05-11

<160> 2

<170> PatentIn version 3.3

<210> 1
<211> 5350
<212> DNA
<213> Sulfolobus islandicus

<220>
<221> misc_feature
<222> (1)..(5350)
<223> Description of sequence: pRN1 plasmid (Fig. 14)

<400> 1
   aattccccat ttcctctaga ctcttctttt tcttctcttc tctctcctgt cttgagtcta 60
   gagtccataa acactgctgc ataacagcat gacaaccgtg ataaagtctg agttcaagat 120
   acaagatatt tataaggagt aggcattttt ttctctcttc ttaaaatatt tttccaatcg 180
   gttatcgata ttctggcact agtgccagaa atttactcaa aagaagtaca aaaattctcg 240
   ccacttggcg agaaatttgc tcaaagtagt gaaacaatat gaaaaagaaa gagaacaatc 300
   acagatatga ttaaactgct gcatgcagcc acgaaatcac aactatgatt atgcagtcca 360
   tatgttaatc ctggtcggat ccgcaaaatt ttagttataa gagttagcta acacgataag 420
   gcaaacagta ttaataaagc gttaatccta cctccaccgt gttatttagc taactttttg 480
   cacgccaaaa gatatttaac agtctgttaa tcctacttta catgggatcc catatgagtg 540
   atctgaagga aaagctaact ctaactcaac taatcctgat tcggctatca aaatcttgtc 600
   aaaccctgga agagttagaa cgatatacag gtgcaaacag aaatgtactt ctcgttacct 660
   tgacacgact ccataaaaaa ggcataatct acaggaaatg gcgtaggttt ggcggtagga 720
   agtatagaga atattgtttg aaaagccgtg acgaactcct gtgaaactcc ccagtttacc 780
   gttattattg atacatatcg atacataatg atacatatgc acacataatg atacagaata 840
   agcgtagaat aatagtataa gcatagtata agcacatgat aagagtataa aataggcgta 900
   aaagcgtacg gctttcagct atatatcccc aaacctacta tttttccacg ctcccttaaa 960
   caaaattcta agataatttg ttttacttta ctgtatatta ttcaatgcat ctaaaatttt 1020
   actgtatacg tctatcgttt tacctgatgc gtataccatt ttcccccatg caccgcattt 1080
   cgtctatata ctcccattct tctatttttt gcacatttca cgcaaaaaac ttcccatacc 1140
   caatcccgcc gtgctttttt tccttttact gcttactctt tcacatagcc ccctctgctt 1200
   ctctaactct gcagttttgc tcaggctttt gcttaatctc tctcacatag ctcttcttct 1260
   ttctgctttt ctaactctac tttctttctt ttcctctact taactactct ttcacttcta 1320
   cttttctaac tcttcttttg ctcttctctt cttagctttg ctaactatgt ttcctttcta 1380
   ctttcattat ctcttcttag cttttgctct ctttctttac tctttatcta cagcttcact 1440
   cttcttcttc tttttccaat ctctgatttt tctcacgatt tctagacaaa ttcccgtaac 1500
   ccccaatccc cccgtgctaa cccaatcttt gattatctta gacaatcttt ccctatgttt 1560
   gactttctta gactaactta gacagtttca aacaaactat tcctacttat gccgacttat 1620
   gccgacttct aacaacatct tacgacatct tatgacatct tacgacatct tacaaatttt 1680
   aggactttca tttctatacc caaactctct gtttttttgc actttcgtgc aaacaattct 1740
   atattccaat tccagactcc tatacccccc atgcttctac tttatcacgc ccgcctttgc 1800
   ttctgtttct tactctcaac tgtttctaac tctgctaact cacaatcccg ctaatcacac 1860
   ttgtgacttt actaactctg cagctttcta actaagcccg ccctgtctaa ctctgctaac 1920
   tatcccccgc cttaatcaca cccgtgattt tgtttttcta actctgcagc tttgcttctt 1980
   acttacagct ttctacctaa ctcttctaat ctctctactt tcctaactct ctgccccccc 2040
   cccccccccc gaacccgacg aaatagaaag ttaattgcag gtattagctt ttcacacggg 2100
   taatactgaa aaatctaccg ttgtcaattt taaggataat tgcggataca attttgatcc 2160
   acaaatgggt agaccataca aactattaaa tggaataaaa ttaggagtct atattccaca 2220
   ggaatggcat gacagattaa tggaaatcgc taaggaaaag aatcttacgt taagtgatgt 2280
   gtgcaggctg gcaattaaag agtatttaga caatcatgat aaacaaaaga agtaaggtaa 2340
   ttcttcatgg aaatgtgaaa aaaacaagaa gaacgggggt gtacatgatt agtttagata 2400
   attcaggtaa taaagatttc tcttcgaatt tctcaagtga gagaattaga tatgctaagt 2460
   ggtttctaga acacggcttt aatattatcc caatcgaccc tgagagcaaa aaacctgtac 2520
   tgaaggagtg gcaaaagtat agtcatgaaa tgccttctga tgaggagaaa cagagatttc 2580
   taaagatgat tgaagagggg tataattacg ctattccagg gggacaaaaa ggactagtga 2640
   ttctcgattt tgagagtaag gagaagttga aagcatggat aggagaatca gcgttagaag 2700
   agctctgtag aaaaacatta tgcacaaaca cggttcatgg cggaatccac atctatgttt 2760
   taagtaacga cattccgcca cacaaaatca atcctctctt tgaagagaat ggtaagggca 2820
   taattgattt acaaagttat aacagttacg ttttaggact tggctcttgt gtgaaccatt 2880
   tacactgtac tactgataaa tgcccgtgga aagaacagaa ctacactact tgttatacat 2940
   tatataatga attaaaggaa attagtaaag ttgacttaaa gagtctccta agattcctgg 3000
   cggaaaaggg caaacgttta ggcataacat taagcaaaac ggcaaaggag tggttagaag 3060
   gaaagaagga agaagaggac acggtggtgg agtttgagga gttgagaaag gaattagtca 3120
   aacgtgacag tgggaaacca gttgagaaaa ttaaagagga gatatgtact aagagcccgc 3180
   ctaaactgat taaggaaata atatgtgaaa acaagactta cgctgatgta aatattgata 3240
   ggagtagagg ggattggcat gttatactct acttaatgaa acacggggtt acggatccag 3300
   acaagatatt ggaattatta ccgcgtgatt caaaagcaaa agaaaatgaa aagtggaata 3360
   cacagaaata ttttgttata actctttcga aagcctggag tgtagtaaag aagtacttag 3420
   aagctaaaag gaaagcacaa aaagacaaaa gcacggctaa ggctctgtta atagaggcga 3480
   ttgctgaaga ggtgttacat gaacattttc tcgttacctt tatacagact gatcaattga 3540
   aggagtcaaa aattgggctt ttcagattca acaagaagaa ggggattttc gaaccatttg 3600
   atgaaagaat tgagaagata attaatgtaa aacttgagga gtataaggaa ttccccctgg 3660
   gttctgataa atcacgtgtg atacgtaata ttaaagaaga gattatgagg agaacacaga 3720
   gactgttatt agaagagtca cttagaattg cgtttcgtaa tgggacactg gaatgggata 3780
   gcaaaggagt aacatggtat gatgttaaag aaagaactcc taaggtgtat tcatttaatt 3840
   atgtggattg gaacttaaag attgaggaga ttgagaagtt taacatgaag gaaataacag 3900
   ttgaagatat agagaattta gcccggcgtg tttgccctag atcacttgag acttttaagc 3960
   agtgggttga tgataaatgg gtgttattgt ttgaagtcat aggctataca ttctatccaa 4020
   agtacatctt taataaggct atacttctca cgggggctgg ggcaaacggg aaatcgactt 4080
   tccttaattt gctgttaaag attctaggac agaagaacgt ttccgcaatg cctttgaaga 4140
   ggatcatgga gagtgataga tttgcttcaa ttgaactatt ccataaacta gcaaatgtaa 4200
   gcagtgagtt gtttgccttt aagatcacaa acacagacct gtttaaaaaa ctcacggggg 4260
   aagactacat agagggacag aagaagttca gagaccccat atacttcatt aattacgcaa 4320
   aactattcaa cgccactaac gaattgcccg tggtttctga tcaaagttat ggattctgga 4380
   gaagatggat tgtgatagaa tttccacacc aattcccgcc ggatcctaac tttttcgata 4440
   agacattcac ggtggaggag gttgaaggag taatcacggt agctgttatc gcattcgccc 4500
   gtgtacttca acagaagaaa ttcgactttg aggatagcag tgctaatgtc aaagaattat 4560
   gggaaagaaa aacagacagt gtttacgcat tcgtgaaaga gttactagaa accgaaaggg 4620
   ctgaatatga tccggcaaac ggcgatttat tcatgcccac tgaggacttc taccaggctt 4680
   atttagaatg gagcgaagag aacgacacaa aagccgaaag caaagccgtg gtaacccaaa 4740
   gattgcaaag caaatttaga ataacaaaag acaagaagaa gattaatgga aaaagagttt 4800
   ggtgttatgt gggaataaga ttgaagaata acaatataag tacggggggc ggacaggacg 4860
   gcgattctcc taattcttta ttagaattat ataaggaatt ccagggcaaa gtggtaagta 4920
   ggaaagactt atatgatatg ctaaagctaa gagcgtttga gttgttggag ttgtgcgaga 4980
   agagaaacat atgtcactgg attgatgaag agcacgtgcg gtttgattga gtccttcaag 5040
   ttttcaattt tttaaattga atttttcatc tgtaatgacc aatttatgtc catagtgtcc 5100
   aacttttttt ctattatttt gatacacggt gggacaataa tataataaaa taatgccttt 5160
   ttagttggac actatggaca taagtagtca cacccgtgat aatatttgta tagtaatggc 5220
   gtttttcaaa tattcttaca acaaaaattt gaatttatct aacgtcattc tctctataga 5280
   accattctct ttatagagct atactttatt cttattaaaa atttagaaat ggggaatttc 5340
   taaaaactgg 5350

<210> 2
<211> 2712
<212> DNA
<213> Sulfolobus islandicus

<220>
<221> gene
<222> (1)..(2712)
<223> Description of sequence: orf904 of pRN1 plasmid

<400> 2
atgataaaca aaagaagtaa ggtaattctt catggaaatg tgaaaaaaac aagaagaacg 60

ggggtgtaca tgattagttt agataattca ggtaataaag atttctcttc gaatttctca 120

agtgagagaa ttagatatgc taagtggttt ctagaacacg gctttaatat tatcccaatc 180

gaccctgaga gcaaaaaacc tgtactgaag gagtggcaaa agtatagtca tgaaatgcct 240

tctgatgagg agaaacagag atttctaaag atgattgaag aggggtataa ttacgctatt 300

ccagggggac aaaaaggact agtgattctc gattttgaga gtaaggagaa gttgaaagca 360

tggataggag aatcagcgtt agaagagctc tgtagaaaaa cattatgcac aaacacggtt 420

catggcggaa tccacatcta tgttttaagt aacgacattc cgccacacaa aatcaatcct 480

ctctttgaag agaatggtaa gggcataatt gatttacaaa gttataacag ttacgtttta 540

ggacttggct cttgtgtgaa ccatttacac tgtactactg ataaatgccc gtggaaagaa 600

cagaactaca ctacttgtta tacattatat aatgaattaa aggaaattag taaagttgac 660

ttaaagagtc tcctaagatt cctggcggaa aagggcaaac gtttaggcat aacattaagc 720

aaaacggcaa aggagtggtt agaaggaaag aaggaagaag aggacacggt ggtggagttt 780

gaggagttga gaaaggaatt agtcaaacgt gacagtggga aaccagttga gaaaattaaa 840

gaggagatat gtactaagag cccgcctaaa ctgattaagg aaataatatg tgaaaacaag 900

acttacgctg atgtaaatat tgataggagt agaggggatt ggcatgttat actctactta 960

atgaaacacg gggttacgga tccagacaag atattggaat tattaccgcg tgattcaaaa 1020

gcaaaagaaa atgaaaagtg gaatacacag aaatattttg ttataactct ttcgaaagcc 1080

tggagtgtag taaagaagta cttagaagct aaaaggaaag cacaaaaaga caaaagcacg 1140

gctaaggctc tgttaataga ggcgattgct gaagaggtgt tacatgaaca ttttctcgtt 1200

acctttatac agactgatca attgaaggag tcaaaaattg ggcttttcag attcaacaag 1260

aagaagggga ttttcgaacc atttgatgaa agaattgaga agataattaa tgtaaaactt 1320

gaggagtata aggaattccc cctgggttct gataaatcac gtgtgatacg taatattaaa 1380

gaagagatta tgaggagaac acagagactg ttattagaag agtcacttag aattgcgttt 1440

cgtaatggga cactggaatg ggatagcaaa ggagtaacat ggtatgatgt taaagaaaga 1500

actcctaagg tgtattcatt taattatgtg gattggaact taaagattga ggagattgag 1560

aagtttaaca tgaaggaaat aacagttgaa gatatagaga atttagcccg gcgtgtttgc 1620

cctagatcac ttgagacttt taagcagtgg gttgatgata aatgggtgtt attgtttgaa 1680

gtcataggct atacattcta tccaaagtac atctttaata aggctatact tctcacgggg 1740

gctggggcaa acgggaaatc gactttcctt aatttgctgt taaagattct aggacagaag 1800

aacgtttccg caatgccttt gaagaggatc atggagagtg atagatttgc ttcaattgaa 1860

ctattccata aactagcaaa tgtaagcagt gagttgtttg cctttaagat cacaaacaca 1920

gacctgttta aaaaactcac gggggaagac tacatagagg gacagaagaa gttcagagac 1980

cccatatact tcattaatta cgcaaaacta ttcaacgcca ctaacgaatt gcccgtggtt 2040

tctgatcaaa gttatggatt ctggagaaga tggattgtga tagaatttcc acaccaattc 2100

ccgccggatc ctaacttttt cgataagaca ttcacggtgg aggaggttga aggagtaatc 2160

acggtagctg ttatcgcatt cgcccgtgta cttcaacaga agaaattcga ctttgaggat 2220

agcagtgcta atgtcaaaga attatgggaa agaaaaacag acagtgttta cgcattcgtg 2280

aaagagttac tagaaaccga aagggctgaa tatgatccgg caaacggcga tttattcatg 2340

cccactgagg acttctacca ggcttattta gaatggagcg aagagaacga cacaaaagcc 2400

gaaagcaaag ccgtggtaac ccaaagattg caaagcaaat ttagaataac aaaagacaag 2460

aagaagatta atggaaaaag agtttggtgt tatgtgggaa taagattgaa gaataacaat 2520

ataagtacgg ggggcggaca ggacggcgat tctcctaatt ctttattaga attatataag 2580

gaattccagg gcaaagtggt aagtaggaaa gacttatatg atatgctaaa gctaagagcg 2640

tttgagttgt tggagttgtg cgagaagaga aacatatgtc actggattga tgaagagcac 2700

gtgcggtttg at 2712

## Description

The present invention relates to an archaeal plasmid vector system comprising: I) one or more selectable marker gene(s) for selection in archaea, and II) a component selected from a) an archaeal origin of replication, b) a homology region to an archaeal chromosome, c) a gene coding for a molecule conferring the ability to integrate the vector into an archaeal chromosome, and d) gene *orf904;* wherein the genes of the plasmid vector do not allow an assembly of a functional virus particle. The present invention also relates to an archaeal plasmid vector system comprising: I) one or more selectable marker gene(s) for selection in archaea, and II) a component selected from a) an archaeal origin of replication, b) a homology region to an archaeal chromosome, and c) a gene coding for a molecule conferring the ability to integrate the vector into an archaeal chromosome; wherein the genes of the plasmid vector do not allow an assembly of a functional virus particle. Further, the present invention relates to a plasmid vector system comprising: I) gene *orf904,* and II) one or more selectable marker gene(s); wherein the vector is non-integrative. Moreover, the present invention relates to host cells transformed with the vector system as well as to kits comprising the vector system or a host cell of the present invention. Furthermore, the present application also relates to a method for producing a protein of interest.

Several documents are cited in this specification. The disclosure content thereof is incorporated herein by reference.

During the last several decades, biotechnology has risen to an important industrial market. One reason for that was the focused research on bacterial species, in particular *E. coli,* and the development of tools for microbiological manipulation thereof. These tools allow e.g. for production of recombinant polypeptides and proteins. However, since *E. coli* represents nowadays a standard tool for microbiological working, other microbiological species like e.g. archaeal species are rarely used as means in biotechnology laboratories or industries. Though, research on archaea such as Sulfolobus is in process and raises possibilities in biotechnology different from those applicable in *E. coli* and provides solutions for disadvantages connected with *E. coli.*

Sulfolobus, a genus of thermoacidophilic crenarchaeotes, has provided much of the information currently available on the physiology and molecular biology of archaea from geothermal environments. Seminal studies of Sulfolobus spp. have addressed, for example, chromatin binding proteins (1;2), replication (3), cell cycle (4), repair (5), transcription (6), translation (7;8) as well as metabolism (9). The genome sequences of three species, *Sulfolobus solfataricus, Sulfolobus tokodaii* and *Sulfolobus acidocaldarius,* have been published (10-12). Microarrays for these organisms are commercially available and proteomic studies have been undertaken (13;14).

On a practical level, these advances reflect the relative ease with which Sulfolobus spp. are manipulated in the laboratory. Sulfolobus cells can be grown aerobically and heterotrophically on a variety of complex and defined carbon sources, either in liquid media or on plates, with doubling times as short as a few hours. Since Sulfolobus spp. are hyperthermophiles with optimal growth temperatures around 80°C, their proteins are intrinsically stable and resistant to proteolysis. As a result, Sulfolobus enzymes expressed in mesophilic hosts can often be purified with the aid of a heat step, which removes most proteins of the host. The structural rigidity of thermophilic proteins also appears to be an advantage for crystallization, which is a prerequisite for X-ray analysis of three-dimensional structure.

Ultimately, however, the comprehensive study of molecular phenomena in any organism requires genetic analysis and manipulation *in vivo.* Although numerous plasmids and viruses have been reported in Sulfolobus spp., the development of these natural genetic elements into experimentally useful tools for Sulfolobus and crenarchaea has lagged behind the corresponding progress made with methanogenic and halophilic archaea (15). Although different plasmid and virus-based vectors have been constructed, (16-21), only SSV1-based viral vectors (20-22) have been successfully applied by other groups to analyze genes *in vivo.* Vectors based on plasmids could not be used reproducibly by other laboratories what represents low utility thereof for industrial application.

One example of a plasmid-based vector is described by Aucelli *et al..* Aucelli *et al.* (16) constructed a shuttle vector based on the sequence of the pSSVx genetic element from *Sulfolobus islandicus.* pSSVx is a hybrid between a plasmid and a Fusellovirus and it is able to spread as a virus satellite in the presence of its helper virus SSV2. In the studies of Aucelli *et al.,* the pSSVx chromosome was fused with an *E. coli* plasmid replicon and the ampicillin resistance gene and used as a vector.

A comparative approach is described in the international patent application WO 2004/106527. This application relates to Sulfolobus expression vectors which comprise genes encoding structural proteins and the site-specific integrase of SSV1, SSV2 or pSSVx. Vectors based on viral sequences are packaged into virus particles - which are generated from structural proteins, e.g. of SSV1 or SSV2 - and spread easily in a host cell culture. Spreading throughout a host cell culture and thus circumventing a stringent selection of transformed cells is the highest advantage of viral-based vectors.

However, working with viral particles in archaeal/bacterial systems is often connected with enormous difficulties in handling thereof. For example, there is a high risk of contamination of different cultures with virus particles.

A further disadvantage is a negative influence of viral sequences on growth behavior of infected host cells. Usually, the presence of viruses decreases the growth and reproduction velocity of an infected host cell. Therefore, in view of a vector system, the replication cycle of virus-based vectors will be generally slower than replication of plasmid-based vectors. Moreover, vectors based on viruses are also disadvantageous due to their large molecular sizes (e.g. the genomes of SSV1 or SSV2 comprise 15.5 kb or 14.8 kb, respectively). Vectors which have a large molecular size constrain the sequences to be incorporated therein (i.e. inserts) to small molecular sizes. Thus, inserts cloned into most viral vectors are limited to shorter nucleic acids. Vectors having a high molecular weight insert vector cannot be packed into infectious virus particles.

Another example of a plasmid-based vector is the shuttle vector pEXSs generated by Cannio et al. (17). The pEXSs vector is composed of an *E. coli* plasmid sequence and the putative SSV1 viral autonomously replicating sequence (ARS). Although reported in 1998, this vector has not been used by any other lab since then, stressing that this vector is of only little practical utility.

In view of the above, the technical problem underlying the present invention was to provide a stable plasmid vector system which is highly efficient in archaea and that may be utilizable for industrial application. The solution to this technical problem is achieved by providing the embodiments characterized in the claims.

Accordingly, the present invention relates to an archaeal plasmid vector system comprising: I) one or more selectable marker gene(s) for selection in archaea, and II) a component selected from a) an archaeal origin of replication, b) a homology region to an archaeal chromosome, c) a gene coding for a molecule conferring the ability to integrate the vector into an archaeal chromosome, and d) gene *orf904;* wherein the genes of the plasmid vector do not allow an assembly of a functional virus particle. The present invention further relates to an archaeal plasmid vector system comprising: I) one or more selectable marker gene(s) for selection in archaea, and II) a component selected from a) an archaeal origin of replication, b) a homology region to an archaeal chromosome, and c) a gene coding for a molecule conferring the ability to integrate the vector into an archaeal chromosome; wherein the genes of the plasmid vector do not allow an assembly of a functional virus particle.

The plasmid vector system of the present invention can be applied for archaea where it is replicated in high levels. The vector system was already used for electroporation and cultivation in Sulfolobus under different conditions whereby reproducible and satisfying results were obtained. Therefore, an industrial application of that vector system is possible.

Due to its low molecular size, sequences of several kb can be cloned into the vector for genetic studies and/or expression.

Since the inventive vector does not carry any genes which might allow the assembly of a functional virus particle, there is no risk of contamination of cell cultures with a virus when using the vector system of the invention. Therefore, the inventive vector system is easy and safe to handle and does not require the precaution that are needed for the handling of virus-based vector systems.

The term "archaea" or "archaeal "refers to a microorganism which represents (besides bacteria and eukaryota) one of the three kingdoms of live. Based on their rRNA, archaea can be divided into two main groups, crenarchaeota and euryarchaeota, and two smaller groups, korarcheota and nanoarcheota.

The term "euryarchaeota", "euryarchaea" or "euryarchaeal" encompasses the classes Archaeglobi, Halobacteria, Methanobacteria, Methanococci, Methanomicrobia, Methanopyri, Thermococci, Thermoplasmata and further unclassified euryarchaeota. According to the invention, thermophilic euryarchaeota such as Thermococci and Thermoplasmata are the preferred euryarchaeota.

The term "crenarchaeota", "crenarchaea" or "crenarchaeal" encompasses the class "Thermoprotei" which comprises, e.g., the orders Desulfurococcales (such as the families Desulfurococcaceae and Pyrodictiaceae), Thermoproteales (such as the families Thermoproteaceae and Thermofilaceae), and Sulfolobales.

The term "Sulfolobales" comprises the family Sulfolobaceae and other unclassified Sulfolobales.

The term "Sulfolobaceae" refers to the genera Acidianus, Metallosphaera, Stygiolobus, Sulfolobus, Sulfurisphaera and other unclassified Sulfolobaceae.

The term "Sulfolobus" refers to hyperthermophilic archaea genera Sulfolobus which comprises, in particular, the species *Sulfolobus acidocaldarius, Sulfolobus brierleyi, Sulfolobus hakonensis, Sulfolobus islandicus, Sulfolobus metallicus, Sulfolobus neozealandicus, Sulfolobus shibatae, Sulfolobus solfataricus, Sulfolobus tengchongensis, Sulfolobus thuringiensis, Sulfolobus tokodaii, Sulfolobus yangmingensis* and cultured *Sulfolobus sp..*

In general, the term "vector" refers to a means for transferring of nucleic acid sequences. The vector of the invention may contain an expression control sequence including, e.g. a promoter, enhancer, silencer, TATA-box, ribosome binding site, attachment sites for transcription factors, terminator, and one or more restriction sites into which an insert sequence can be cloned. The vector may have a size of 1 kb to 50 kb, preferably 2 to 30 kb, more preferably 3 to 20 kb, and most preferably 3 to 10 kb.

The term "plasmid" refers to a circular, extrachromosomal nucleic acid molecule capable of replication in a cell.

The term "plasmid vector system" refers to a vector system which is based on a plasmid or parts thereof. In the context of the present invention, the term "plasmid vector system" is synonymously used to the terms "plasmid vector" or "vector".

The term "archaeal plasmid vector system" refers to a vector system as defined above which is functional in archaea, i.e. can be applied for archaea, for example, for cloning and/or expression of genes in archaea. The vector system is preferably functional in crenarchaea, more preferably Sulfolobales, even more preferably Sulfolobaceae and most preferably Sulfolobus. Also preferably, the vector system is functional euryarchaeota, more preferably thermophilic euryarchaeota and most preferably Thermococci and/or Thermoplasmata.

The term "selectable marker gene" refers to a gene encoding a protein or an RNA that, when expressing said gene in a cell, confers a phenotype onto the cell which allows a selection of the cell expressing said selectable marker gene. The selectable marker gene does not naturally occur on the plasmid on which the plasmid vector is based. Preferably, the selectable marker gene was introduced into the vector by recombinant biochemical methods, e.g. via cloning.

The selectable marker gene allows for selection of cells which were transformed with the vector carrying the selectable marker gene. Preferably, the selectable marker gene encodes a protein that provides for synthesis of a compound necessary for the cell metabolism under natural or modified environment conditions or which, in general, allows the cell to survive under said conditions. More preferably, the selectable marker gene is an essential gene or a gene conferring a growth advantage for the cell.

The selectable marker gene can encode a protein that confers resistance to an antibiotic on said cell. Antibiotics can be divided into several subclasses according to their structural elements or function on the cell treated therewith: aminoglycoside, arsenic-based, bacteriostatic, β-lactam, fluoroquinolone, glycopeptide, polyketide, and sulfonamide antibiotics. With respect to the invention, the selectable marker gene, preferably, confers resistance to all kinds of antibiotics. More preferably, the selectable marker gene confers resistance to the antibiotics selected from the group consisting of ampicillin, carbenicillin, penicillin (G), cephalosporins, cefotaxim, cefalexin, vancomycin, cycloserine, chloramphenicol, erythromycin, lincomycin, tetracyclin, spectinomycin, clindamycin, chlortetracycline, gentamycin, hygromycin (B), kanamycin, neomycin, streptomycin, G418, tobramycin, rifampicin, mitomycin (C), nalidixic acid, doxorubicin, 5-flurouracil, 6-mercaptopurine, micanozole, trimethoprim, methotrexat, metronidazole, sulfmetoxazole, benylpenicillin, propicillin, azidocillin, flucloxacillin, dicloxacillin, novobiocin and oxacillin. Selection of cells which were transformed with a vector carrying the antibiotic selectable gene and are therefore resistant to that antibiotic is performed using an antibiotic-containing medium for cultivation of those cells.

Further, the selectable marker gene may encode a protein which influences the growth behavior of a cell when expressed inside this cell under the presence of an inhibitor or activator/enhancer of said protein. The influence can be in a positive or negative manner, whereby the positive manner is preferred. Preferably, the protein encoded by the selectable marker gene influences the cell, in the presence of an inhibitor, in a positive manner. More preferably, the selectable marker gene is the 3-hydroxy-3-methylglutaryl coenzyme A (HMG-CoA) reductase gene (44), most preferably, the selectable marker gene is a thermostable HMG-CoA reductase. Both eukaryotes and archaebacteria use 3-hydroxy-3-methylglutaryl coenzyme A (HMG-CoA) reductase to synthesize mevalonate, which eukaryotes employ in the production of sterols and archaea need for the isoprenoid side chains of their unique and characteristic lipids. Preferred inhibitors for HMG-CoA are statins selected from the group consisting of simvastatin, mevinolin, lovastatin, atorvastatin and pravastatin and analogues thereof.

Also preferably, the selectable marker gene provides for the synthesis of a metabolic compound. More preferably, the selectable marker gene encodes substances which are necessary for formation of nucleotides (adenine (A), guanine (G), cytosine (C), uracil (U) or thymine (T)) or amino acids (alanine (A, Ala), arginine (R, Arg), asparagine (N, Asn), aspartic acid (D, Asp), cysteine (C, Cys), glutamate (G, Glu), glutamine (Q, Gln), glycine (G, Gly), histidine (H, His), isoleucine (I, lle), leucine (L, Leu), lysine (K, Lys), methionine (M, Met), phenylalanine (F, Phe), proline (P, Pro), serine (S, Ser), threonine (T, Thr), tryptophan (W, Trp), tyrosin (Y, Tyr) and valine (V, Val)) and modification thereof.

Of those selectable marker genes, which provide for the synthesis of a metabolic compound, genes encoding orotidine-5'-monophosphate pyrophosphorlyase and/or orotidine-5'-monophosphate decarboxylase are especially preferred. Orotidine-5'-monophosphate pyrophosphorlyase (PyrE) and orotidine-5'-monophosphate decarboxylase (PyrF) are necessary for the synthesis of uracil. Both genes can be used in vectors for transformation of uracil-auxotrophs, e.g. cells which do not carry functional *pyrE* and/or *pyrF* genes, neither on their chromosome nor on their plasmids (26). Selection of cells which were transformed with a vector carrying either or both genes of *pyrE and pyrF* genes is performed using uracil-free mediums for cultivating cells.

In accordance with the invention, any of the above described selectable marker genes may be used for selection in archaea.

An origin of replication may also be used as a selectable marker gene enabling propagation of a plasmid vector.

Further, a reporter gene may be used as a selectable marker gene. Preferred reporter genes are genes encoding β-galactosidase, luciferase, green fluorescent protein or peptides and proteins which preferably bind or interact with reporter molecules such as fluorescent dyes, as well as variants thereof.

In one alternative of the present invention, the vector comprises also an archaeal origin of replication.

The term "origin of replication" or "replication origin" refers to a DNA region that is essential for starting its replication.

The term "archaeal origin of replication" refers to a DNA region which is essential for starting its replication in archaea. In general, any replication origin which can start a replication in archaea may be used in connection with the present invention.

Many archaeal species are known to incorporate one to three origins of replication in their chromosomes (*oriC*). Further, archaea also contain a variety of plasmids which carry origins of replication. For example, the crenarchaea Sulfolobus incorporates several plasmids. In detail, these are the two types of plasmids: 1) rather small cryptic plasmids with genome size of 5-14 kb and 2) a family of conjugative plasmids with genomes larger than 25 kb (28;47). Examples of crenarchaeal plasmids of type 1) are plasmids of the pRN family such as pRN1, pRN2, pSSVx, pDL10, pHEN7, pXQ1 and pST1; pIT3, pTAU4, pORA1 and pTIK4 and variants thereof. Plasmids of the pING family (e.g. pING to pING4 and pING6), pTC, pNOB8, pKEF9, pHVE14 and pARN4 are examples of conjugative (type 2) plasmids. Further, archaea harbor various classes of viruses such as Siphoviridae, Myoviridae, Salterprovirus, SH1, Rudiviridae, Lipothrixviridae, Guttaviridae, Globuloviridae, Ampullaviridae, Bicaudaviridae, Fuselloviridae (e.g. SSV1, SSV2, SSV-K1 and SSV-RH) and STIV.

Thus, the origin of replication may be an archaeal chromosomal, archaeal viral or archaeal plasmidal replication origin. Preferably, the origin of replication is an archaeal chromosomal or archaeal plasmidal replication origin.

The archaeal chromosomal origin of replication may be a chromosomal crenarchaeal origin of replication, preferably an origin of replication of a member of the class Thermoprotei, more preferably of a member of the order Sulfolobales, even more preferably of a member of the family Sulfolobaceae and most preferably of a member of the genus Sulfolobus. Preferably, a member of the genus Sulfolobus is selected from the group consisting of *Sulfolobus acidocaldarius, Sulfolobus islandicus, Sulfolobus metallicus, Sulfolobus neozealandicus, Sulfolobus shibatae, Sulfolobus solfataricus, Sulfolobus tengchongensis, Sulfolobus thuringiensis, Sulfolobus tokodaii, Sulfolobus yangmingensis* and other cultured *Sulfolobus sp..* The archaeal chromosomal origin of replication may also be a chromosomal euryarchaeal origin of replication, preferably an origin of replication of a thermophilic euryarchaeota, more preferably of Thermococci or Thermoplasmata.

The archaeal plasmidal origin of replication may be selected from replication origins of any known archaeal plasmid, preferably from replication origins of the pRN plasmid family, or from replication origins of the plasmids of type 1) as defined above, more preferably from replication origins of the pRN plasmids, even more preferably from replication origins of the pRN1 plasmids and most preferably from replication origins of the pRN1 plasmid from *Sulfolobus islandicus.*

All plasmids of the Sulfolobus pRN family share three conserved open reading frames (ORFs). For one member of this family, plasmid pRN1, those three ORFs are named *orf56, orf80,* and *orf904.* Up to now, no pRN plasmid has been discovered outside the crenarchaeal phylum. Moreover, plasmids of the pRN family are not related to any other known eubacterial or archaeal plasmid. The first pRN plasmid which was completely sequenced was plasmid pRN1 isolated from the *Sulfolobus islandicus* strain RENH1 (41). A more detailed description of the pRN1 plasmid is given below.

The term "homology region to an archaeal chromosome" of a second alternative of the present invention relates to a region on the plasmid vector which shows homology to a region on the chromosome of an archaeon. The archaeon is preferably a crenarchaeon preferably selected from a member of the class Thermoprotei, more preferably a member of the order Sulfolobales, even more preferably a member of the family Sulfolobaceae and most preferably a member of the genus Sulfolobus. Preferably, the member of the genus Sulfolobus is selected from the group consisting of *Sulfolobus acidocaldarius, Sulfolobus islandicus, Sulfolobus metallicus, Sulfolobus neozealandicus, Sulfolobus shibatae, Sulfolobus solfataricus, Sulfolobus tengchongensis, Sulfolobus thuringiensis, Sulfolobus tokodaii, Sulfolobus yangmingensis* and other cultured *Sulfolobus sp..* Also preferably, the archaeon is an euryarchaeon, more preferably a thermophilic euryarchaeota, most preferably Thermococci or Thermoplasmata.

The homology region may comprise a length of 10-50,000 bp, preferably 30-10,000 bp, more preferably 50-5,000 bp and most preferably 100-1,000 bp. The region on the plasmid vector may differ from the chromosomal sequence in one or more mutation(s), such as one or more substituted, inserted and/or deleted nucleic acid(s), and show a sequence homology of at least 60%, preferably at least 80%, more preferably at least 85%, equally more preferably at least 90%, even more preferably at least 95% and most preferably at least 97%, to the chromosomal sequence.

In order to determine the percentage to which two sequences (nucleic acid or amino acid sequences) are identical, the sequences can be aligned and compared to each other. Therefore, gaps can be inserted into one sequence at that positions where the sequence lacks a homolog at a sequence which is compared thereto. If a position in the first sequence is occupied by the same compound as is the case at a position in the second sequence, the two sequences are identical at this position. The percentage to which two sequences are identical is a function of the number of identical positions divided by the total number of positions.

The percentage to which two sequences are identical can be determined using a mathematical algorithm. A preferred, but not limiting, example of a mathematical algorithm which can be used is the algorithm as described by Karlin *et al.* (45) and Altschul *et al.* (46). Such an algorithm is integrated in the BLAST program. Sequences which are identical or similar to the sequences of the present invention to a certain extent can be identified by this program.

The homology region on the vector enables integration of the vector into the archaeal chromosome. The vector may be integrated in a stable way and may or may not be removed again from the chromosome. If the vector is removed again, the excision sites may be the same or different compared to the sites for integration of the vector. Thus, the vector may be excised completely or only in part. Further, additional parts of the chromosome may be removed together with the (full length or partial) vector. The integration process may occur via recombination of homologous sequences on the vector and chromosome. Preferably, the integration takes place via crossover, more preferably via single crossover.

In a third alternative of the present invention, the term "molecule conferring the ability to integrate the vector into an archaeal chromosome" refers to a molecule which is capable of catalyzing the integration of DNA into a specific site in the archaeal chromosome. Preferably, the molecule is an integrase or a transposase. In general, any integrase and transposase molecule is comprised by the present invention as long as it catalyses the integration of the vector into the chromosomal DNA of an archaeal cell. Integrases are enzymatic proteins which are naturally present in retroviruses or retrotransposons and which catalyze integration of the nucleic acid thereof into the host chromosome. More preferably, the integrase molecule is an integrase from an archaeal virus selected from Fuselloviridae, even more preferably from the viruses SSV1, SSV2, SSV-K1 and SSV-RH and related viruses. Also preferably, the integrase molecule is an integrase from the conjugative plasmids, preferably of the PING family (e.g. plNG1 to pING4 and piNG6), pTC, pNOB8, pKEF9, pHVE14 and pARN4 plasmids. Integrases from other archaeal viruses are also preferred. The attachment site for integration may be located within the coding sequence of the integrase gene on the vector or outside in the remaining nucleotide sequences on the vector. Preferably, the attachment site is located inside the coding sequence, whereby a re-excision of the gene form the chromosome can be prevented leading to stable integration.

Transposases are enzymes which cut out DNA from one location and insert this DNA at another location. Preferably the transposase molecule is an archaeal transposase. The transposase molecule can be a transposase from a crenarchaeaota, preferably from a member of the class Thermoprotei, more preferably from a member of the order Sulfolobales, even more preferably from a member of the family Sulfolobaceae and most preferably from a member of the genus Sulfolobus. Also preferably, the transposase molecule is a transposase from the same taxonomic order as the host cell. Further preferably, the transposase molecule is sequence-specific for the chromosomal sequence enabling the construction of cells with defined integration sites.

Integration of the vector into the chromosome ensures a stable remaining and replication of the vector in the cell. For example, the vector may encode a specific protein which has to be replicated over many cycles of cell division. Integration of the vector into the chromosome ensures that the coding sequence is inherited to the following cell generations, even without selection for said vector. These cells can be grown in rich and optimized media allowing for rapid growth.

Finally, the term "gene *orf904"* of the fourth alternative of the present invention refers to the open reading frame sequence *orf904* in the pRN plasmid family of Sulfolobus, preferably the pRN plasmid of Sulfolobus, more preferably the pRN1 plasmid of Sulfolobus and most preferably the pRN1 plasmid of *Sulfolobus islandicus.* According to the invention, this term also encompasses gene sequences which differ in one or more mutation(s), such as one or more substituted, inserted and/or deleted nucleic acid(s), and show, over a stretch of about 300 amino acids, a sequence homology of at least 60%, preferably at least 70%, more preferably at least 80%, equally more preferably at least 85%, even more preferably at least 90% and most preferably at least 97%, to the gene *orf904* of the pRN1 plasmid of Sulfolobus. Methods for determination of sequence homology are described above. The multifunctional protein orf904 appears to be the sole essential gene for plasmid replication of the pRN1 plasmid and it could carry out the replication initiation function. The inventors discovered the importance of *orf904* for construction of self replicating vector systems. A detailed description of *orf904* is given below.

The present invention provides an archaeal plasmid vector system. The vector system comprises one or more selectable marker gene(s) for selection in archaea. The vector system further comprises an archaeal component and/or a component which is functional in archaea, respectively. In particular, these components provide for replication of the vector in a host cell. In one alternative, the vector system comprises an archaeal origin of replication (a). In another alternative, the vector system incorporates the gene *orf904* coding for a replication protein (d). Both components enable the vector to be replicated within an archaeal cell without being integrated into an archaeal chromosome. In further alternatives, the vector comprises a homology region to an archaeal chromosome (b) or a gene coding for a molecule conferring the ability to integrate the vector into an archaeal chromosome (c). As described above, those sequences allow for stable integration of the plasmid vector into an archaeal chromosome which leads to replication of the vector in an integrated form. According to the present invention, the vector can comprise the selectable marker gene(s) and any one of the components (a), (b), (c) or (d). Thus, the vector may comprise the selectable marker gene(s) and component (a), the selectable marker gene(s) and component (b), the selectable marker gene(s) and component (c) or the selectable marker gene(s) and component (d), in specific embodiments. The vector may also comprise two, three or four of those components together with the selectable marker gene(s). For example, the vector may comprise the selectable marker gene(s) and components (a) and (b); (a) and (c); (a) and (d); (b) and (c); (b) and (d); (c) and (d); (a), (b) and (c); (a), (b), and (d); (a), (c) and (d); (b), (c) and (d); or (a), (b), (c) and (d) together, in specific embodiments according to the present invention.

A further characteristic feature of the plasmid vector according to the present invention is that the genes of the plasmid vector do not allow an assembly of a functional virus particle. The term "functional virus particle" refers to a virus particle which is capable of encapsulating or integrating a nucleic acid molecule in any possible way. Preferably, the plasmid vector does not contain any sequences which allow for formation of virus or virus-like particles. Thus, the vector sequence (as DNA or RNA) cannot be encapsulated into virus or virus-like particles and be spread as a virus. More preferably, the plasmid vector does not contain any sequences which could lead to formation of virus or virus-like particles, solely or in combination with helper components. For example, the plasmid-virus hybrid pSSVx is not able to form virus or virus-like particles on its own. However, in combination with its helper plasmid SSV2, pSSVx generates virus-like particles. Even more preferably, the plasmid vector of the present invention does not contain any structural genes of viruses and/or any genes encoding components necessary for packaging of nucleic acids (e.g. packaging signals which are capable of directing nucleic acid molecules into the envelope of a virion). Most preferably, the plasmid vector neither comprises the above genes in their natural forms, i.e. without mutations, nor any mutated forms (including deletions, insertions or substitutions) of those genes.

In one preferred embodiment, the plasmid vector of the present invention only comprises viral genes selected from the group consisting of viral integrases and viral origins of replication.

The invention is based on the finding that vectors based on the sequence of Sulfolobus plasmids are stable and effective, especially for use in Sulfolobus. In particular, vectors based on the pRN1 plasmid, a plasmid from the pRN family of *Sulfolobus islandicus,* were found to be very advantageous.

The pRN1 plasmid is notable for its relatively small size (5.4 kb), copy numbers ranging from 2 to 20 in mid-log phase, and the three genes (coding for orf56, orf80, and orf904) which are also are conserved in other Sulfolobus plasmids. In previous works, the inventors have analyzed the three conserved proteins (representing two DNA-binding proteins and the replication protein) and the transcriptional activity of the plasmid (23-25). Those studies revealed that orf56 binds upstream of its own gene and down-regulates the expression of the cotranscript *orf56*/*orf904.* It therefore appears that orf56 could be involved in regulating the copy number of the plasmid. Similarly, orf80 is a sequence-specific DNA-binding protein, but the physiological function of this protein has remained unclear. *Orf904* encodes the third conserved protein, a multifunctional 110 kDa replication enzyme that appears to play a central role in replication. The gene *orf904* is by far the largest ORF of the pRN plasmid family. The corresponding genes occupy about a third to a half of the plasmids and encode proteins of about 900-1,000 amino acids. Within the C-terminal part of the proteins a helicase domain of superfamily 3 can be identified. The best-studied protein containing this domain is the bacteriophage P4 α protein, which is a multifunctional replication protein harboring a helicase domain in its C-terminal part. The N-terminal 40 amino acids are not found in the corresponding proteins of the other plasmids.

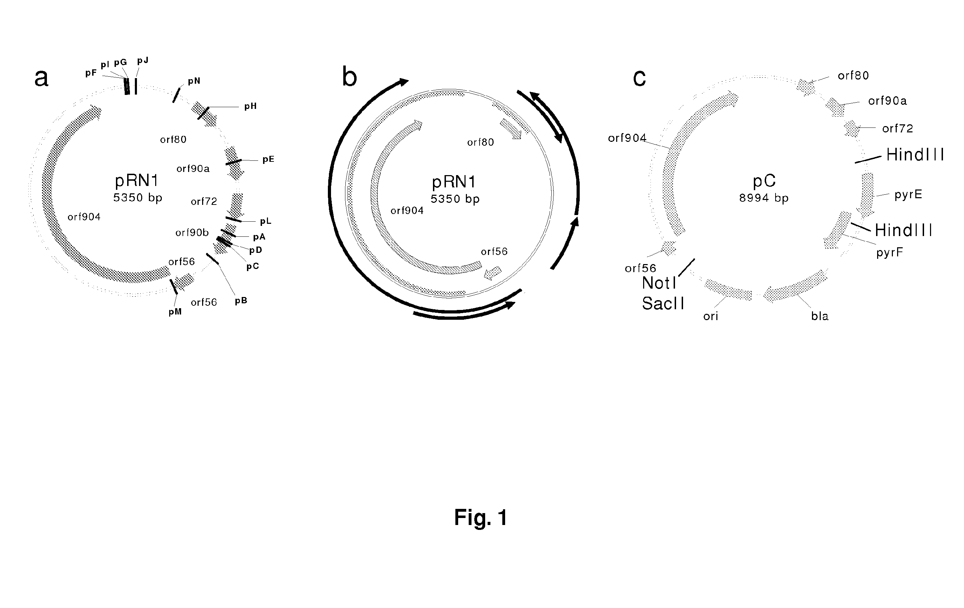

In view of the vectors based on the pRN1 plasmid, especially *E. coli*-Sulfolobus shuttle vectors, the inventors took an empirical approach, in which an artificial transposon containing *E. coli* elements required for shuttle function was inserted at many locations around pRN1 (Fig. 1 A). The resulting plasmids provided a series of potential shuttle vectors differing only in the relative location and orientation of inserted genes, from which the best-performing constructs were identified (Table 1). The series also provided a way to reveal experimentally which regions of pRN1 may be important for successful propagation in a variety of host cells, e.g. Sulfolobus cells. A detailed description of assays performed in the context of this series is provided by Examples 1 to 3 below.

As a result, the inventors developed a variety of stable vectors based on the sequence of pRN1. Further, the inventors also found out that *orf904* and the replication operon *orf56*/*orf904* are essential for construction of stable plasmids. However, neither *orf80* nor the remaining open reading frames, *orf72, orf90a* or *orf90b,* are essential for plasmid replication.

In a second approach, the inventors developed a series of vectors which are also based on the pRN1 sequence. In contrast to the vectors described above, however, a different system was used as a selectable marker gene - HMG-CoA reductase instead of pyrEF - (Fig. 11-13).

In a further approach, the inventors developed vectors ("pD-suicide") comprising a homology region to an archaeal chromosome (Fig. 7). As shown in Fig. 8 and 9, the vectors can be stably integrated into an archaeal chromosome via single crossover. Further, the vectors may be removed from the chromosome via recombination (Fig. 10).

As described above, the inventors realized the importance of *orf904* and the replication operon *orf56*/*orf904* as components for construction of stable vectors.

Therefore, the present invention also relates to a plasmid vector system comprising: I) gene *orf904,* and II) one or more selectable marker gene(s); wherein the vector is non-integrative.

The terms "gene *orf904"* and "selectable marker gene" have been defined above.

The term "non-integrative" refers to a feature of the plasmid vector wherein the vector is not integrated into a chromosomal DNA of a cell. A non-integrative vector is characterized by the capability to replicate in a cell without being integrated into its chromosome.

In one specific embodiment of the present invention, the vector systems comprise one or more selectable marker gene(s) which is/are essential gene(s) or gene(s) conferring a growth advantage for the cell.

In another specific embodiment, the vector systems comprise a selectable marker gene(s) selected from the genes coding for orotidine-5'-monophosphate pyrophosphorlyase and/or orotidine-5'-monophosphae decarboxylase, or HMG-CoA reductase.

Based on the results with various constructs and recipient strains, the inventors realized that creation of a suitably reliable selection is very important for establishing stably replicating vectors, in particular derived from the Sulfolobus plasmid pRN1. For example, problems related to reversion of the uracil-auxotrophy could be avoided by the use of a *pyrE* deletion mutant of *S. acidocaldarius.* These deletion mutants could be easily selected for transformation with a plasmid carrying genes coding for orotidine-5'-monophosphate pyrophosphorlyase. In addition to the PyrEF selection, selection using genes encoding HMG-CoA reductase turned out to be advantageous for selection in archaea.

The inventive vector systems may be applied in a variety of biochemical and biotechnological fields. For example, the vector systems can be used in connection with genetic studies, especially on archaeal species. With respect to such studies, the vector is integrated into the archaeal chromosome or replicated episomally. The integration can be carried out either via integrases, transposases and/or homology regions as described above. Further, the vector system can be also applied for expression of proteins of interest (i.e. as expression vectors) or as shuttle vectors for transfer of genes between different organisms.

In one embodiment of the present invention, the vector is a cloning vector. Preferably, the vector serves for cloning of genes into archaea, preferably crenarchaeota, more preferably Sulfolobales, even more preferably Sulfolobaceae and most preferably Sulfolobus. Most preferably, the vector serves for cloning into *Sulfolobus acidocaldarius, Sulfolobus brierleyi, Sulfolobus hakonensis, Sulfolobus islandicus, Sulfolobus metallicus, Sulfolobus neozealandicus, Sulfolobus shibatae, Sulfolobus solfataricus, Sulfolobus tengchongensis, Sulfolobus thuringiensis, Sulfolobus tokodaii, Sulfolobus yangmingensis and Sulfolobus sp..* Also preferably, the vector serves for cloning into euryarchaeota, more preferably thermophilic euryarchaeota, most preferably Thermococci and/or Thermoplasmata.

In another embodiment, the vector is an expression vector. An expression vector includes an expression cassette comprising a promoter, a ribosome biding site, a terminator and a gene which has to be expressed. The gene which has to be expressed is a gene of interest encoding a protein or polypeptide of interest. The substance (e.g. gene, protein or peptide) of interest may be naturally occurring or modified, e.g. having a mutated sequence as compared to a naturally occurring substance. The substance of interest may also be modified by comprising a fusion such as a purification tag.

Preferably, the expression vector serves for expression in archaea. More preferably, the expression vector serves for expression in crenarchaea, still more preferably Sulfolobales, even more preferably Sulfolobaceae and most preferably Sulfolobus. Preferably, the expression vector serves for expression in Sulfolobus selected from the group consisting from *Sulfolobus acidocaldarius, Sulfolobus brierleyi, Sulfolobus hakonensis, Sulfolobus islandicus, Sulfolobus metallicus, Sulfolobus neozealandicus, Sulfolobus shibatae, Sulfolobus solfataricus, Sulfolobus tengchongensis, Sulfolobus thuringiensis, Sulfolobus tokodaii, Sulfolobus yangmingensis and Sulfolobus sp..* Also preferably, the expression vector serves for expression in euryarchaeota, more preferably in thermophilic euryarchaeota, most preferably in Thermococci and/or Thermoplasmata.

In a further embodiment, the vector is a shuttle vector. The term "shuttle vector" refers to a vector able to replicate in two different organisms. Preferably, the shuttle vector is able to replicate in an archaeal and a bacterial or eukaryotic (such as yeast) organism. More preferably, the shuttle vector is able to replicate in an archaeal and a bacterial organism. Most preferably, the shuttle vector is able to replicate in an archaeal organism and in *E. coli.* Among archaeal organisms, crenarchaea are preferred, Sulfolobales are more preferred, Sulfolobaceae are even more preferred and Sulfolobus species are mostly preferred. Most preferably, the shuttle vector is able to replicate in *E. coli* and *Sulfolobus acidocaldarius, Sulfolobus brierleyi, Sulfolobus hakonensis, Sulfolobus islandicus, Sulfolobus metallicus, Sulfolobus neozealandicus, Sulfolobus shibatae, Sulfolobus solfataricus, Sulfolobus tengchongensis, Sulfolobus thuringiensis, Sulfolobus tokodaii, Sulfolobus yangmingensis and Sulfolobus sp..* Also preferably, the shuttle vector is able to replicate in *E. coli* and in euryarchaeota, more preferably in thermophilic euryarchaeota, most preferably in Thermococci and/or Thermoplasmata.

According to the invention, in the second organism such as *E. coli,* vector replication occurs extrachromosomally as a stable plasmid. In contrast, in archaea, vector replication can either take place extrachromosomally or integrated into the chromosome. Shuttle vectors are used to transfer DNA cloned therein between different organisms. For example, a shuttle vector may be used in one organism for efficient replication and can be transformed into another organism for expression of genes of interest.

According to the invention, genes of several kb in length can be cloned into the inventive vectors. When applied for expression of genes of interest, the use of the inventive vectors may lead to production of proteins of high molecular weights.

In another embodiment of the invention, the vector further comprises an *E. coli* origin of replication. Presence of a second replication origin leads to formation of shuttle vectors as described above.

The term *"E* coli origin of replication" refers to a DNA region which is essential for starting its replication in *E coli.* Among the originally occurring origin of replication in *E. coli,* any origin of replication which is functional in *E. coli* can be used in accordance with the present invention, i.e. origins of other bacterial or non-bacterial species are also encompassed by the present invention. Further, an archaeal plasmid vector system comprising a non-*E. coli* origin of replication, in particular bacterial non-*E. coli* origin of replication, can also be constructed using the instructions provided herein.

According to the invention, a vector system can be initially constructed which incorporates a further origin of replication (e.g. an *E. coli* replication origin). Then, the origin of replication can be removed from the vector again using e.g. cloning methods known in the art.

In one embodiment, the vector comprises one or more selectable marker gene(s) for selection in *E. coli.*

Any of the selectable marker gene(s) as defined previously can be used in accordance with the present invention. Further, any selectable marker gene for selection in *E. coli* known in the art can be applied for selection. Preferably, the selectable marker gene for selection in *E. coli* is a gene conferring antibiotic resistance. Also preferably, a gene encoding a reporter molecule is used as a selectable marker gene.

In a further embodiment of the invention, the vector system comprises an additional expression cassette for a reporter molecule.

The term "reporter molecule" refers to a protein or peptide which visibly indicates ("reports") its presence in a cell or in a certain compartment of a cell. Reporter molecules are well known in the art. A reporter molecule can report its presence with or without interaction with other substances. For example, the reporter molecule can undergo a reaction with another substance which leads to a change of color. Further, the reporter molecule can interact with a colorful or fluorescent dye or a radioactive substance for indication. According to the invention, a reporter molecule can also be used for detection of successful transformation of a cell.

The reporter molecule is preferably selected from the group consisting of β-galactosidase, luciferase, green fluorescent protein and variants thereof.

In one specific embodiment, the plasmid vector system comprises one or more selectable marker gene(s) for selection in archaea selected from essential gene(s) for a host organism and gene(s) conferring a growth advantage, and an archaeal origin of replication, wherein the archaeal origin of replication is an archaeal plasmidal origin of replication; wherein the genes of the plasmid vector do not allow an assembly of a functional virus particle.

In another specific embodiment, the plasmid vector system comprises one or more selectable marker gene(s) for selection in archaea selected from the genes coding for orotidine-5'-monophosphate pyrophosphorlyase and/or orotidine-5'-monophosphate decarboxylase, or HMG-CoA reductase, and an archaeal origin of replication, wherein the archaeal origin of replication is an origin of replication of the pRN1 plasmid; wherein the genes of the plasmid vector do not allow an assembly of a functional virus particle.

In a further specific embodiment, the plasmid vector system comprises one or more selectable marker gene(s) for selection in archaea selected from essential gene(s) for a host organism and gene(s) conferring a growth advantage, and a homology region to an archaeal chromosome; wherein the genes of the plasmid vector do not allow an assembly of a functional virus particle.

In another specific embodiment, the plasmid vector system comprises one or more selectable marker gene(s) for selection in archaea selected from the genes coding for orotidine-5'-monophosphate pyrophosphorlyase and/or orotidine-5'-monophosphate decarboxylase, or HMG-CoA reductase, and a homology region to an archaeal chromosome, wherein the homology region to an archaeal chromosome is 10 to 50,000 bp, preferably 30 to 10,000 bp, more preferably 50 to 5,000 bp and most preferably 100 to 1,000 bp in length; wherein the genes of the plasmid vector do not allow an assembly of a functional virus particle.

In a further specific embodiment, the plasmid vector system comprises one or more selectable marker gene(s) for selection in archaea selected from the genes coding for orotidine-5'-monophosphate pyrophosphorlyase and/or orotidine-5'-monophosphate decarboxylase, or HMG-CoA reductase, and a homology region to an archaeal chromosome, wherein the homology region to an archaeal chromosome is a homology region of a crenarchaeal chromosome, preferably a chromosome of a member of the class Thermoprotei, more preferably of the order Sulfolobales, even more preferably of the family Sulfolobaceae and most preferably of the genus Sulfolobus; wherein the genes of the plasmid vector do not allow an assembly of a functional virus particle.

In addition, it is also preferred that the vector system of the specific embodiments described above comprises an *E. coli* origin of replication and one or more selectable marker gene(s) for selection in *E. coli,* in further embodiments.

The present invention also relates to a host cell transformed with the vector of the invention.

Preferably, the host cell is an archaeal cell. An archaeal cell may be a crenarchaeal cell, preferably a cell of a member of the class Thermoprotei, more preferably of a member of the order Sulfolobales, even more preferably of a member of the family Sulfolobaceae and most preferably of a member of the genus Sulfolobus. Preferably, the member of the genus Sulfolobus is selected from the group consisting of *Sulfolobus acidocaldarius, Sulfolobus islandicus, Sulfolobus metallicus, Sulfolobus neozealandicus, Sulfolobus shibatae, Sulfolobus solfataricus, Sulfolobus tengchongensis, Sulfolobus thuringiensis, Sulfolobus tokodaii, Sulfolobus yangmingensis* and other cultured *Sulfolobus sp..* Also preferably, the archaeal cell is an euryarchaeal cell, preferably a cell of a thermophilic euryarchaeota, more preferably of Thermococci and/or Thermoplasmata.

Further preferably, in particular for transformation with a shuttle vector according to the invention, the host cell is a bacterial cell, preferably an *E. coli* cell, or an eukaryotic cell, preferably a yeast cell. Preferred *E. coli* strains are BL21, BL21 (DE3) or BL21 (DE3) pLysS and BL21, HB101, JM109, XL-1blue, DH10B, DH12S, DH5alpha, DB3.1, Stb14, TOP10 and derivatives thereof.

In a further embodiment of the present invention, the host cell produces a protein of interest.

The vectors of the present invention can be introduced into the host cell using the same techniques as those used for introduction of common plasmid vectors. Examples of such techniques are electroporation, calcium phosphate-DNA transfection, liposome, and immunogene technique and the like.

The present invention also relates to a method of producing a protein of interest comprising culturing the host cell of the present invention under suitable conditions and isolating said polypeptide from the cells or the cell culture supernatant. The present invention is also directed to the use of the vector system for production of a protein of interest.

Moreover, the present invention relates to a kit comprising a vector system or a host cell of the present invention in one or more container(s).

The Figures indicate:
Fig. 1 shows physical maps of vectors. In A, positions of the insertion sites of the *E. coli* replicon and the *pyrEF* marker genes for shuttle vectors pA-pN are indicated. B shows conserved features of pRN1: crosshatched arrows: conserved open reading frames, grey area: conserved on the nucleotide level within the pRN family plasmids, black arrows: transcripts. C is a vector map of the shuttle construct pC.

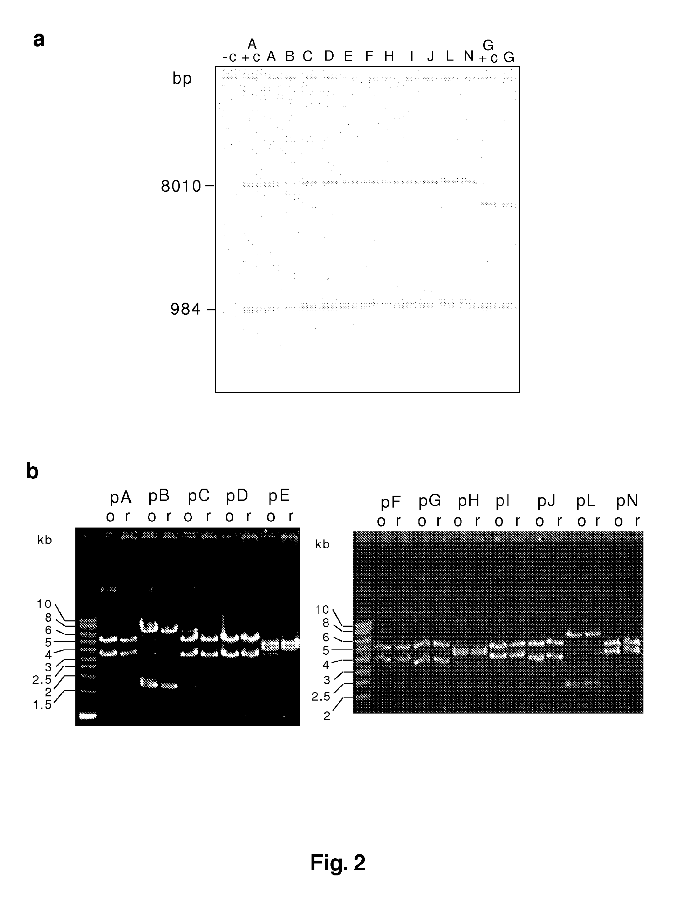

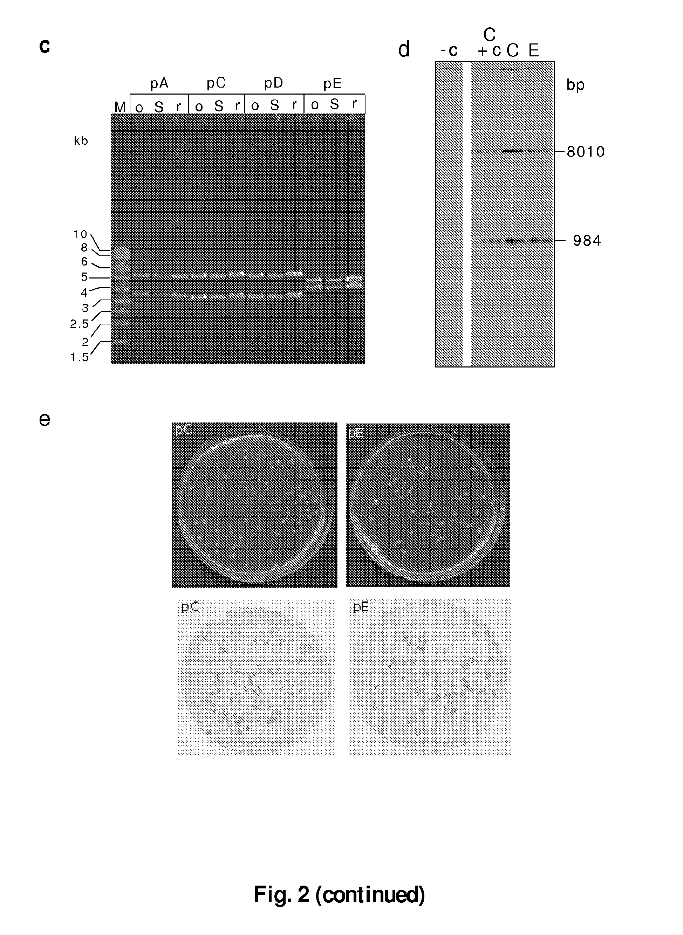

Fig. 2 is directed to the analysis of *S*. *acidocaldarius* transformants. A shows a Southern blot of *Hind*III digested genomic DNA preparations (-c: untransformed MR31, A+c: positive control: plasmid pA from *E. coli,* G+c: separate positive control (plasmid pG from *E. coli*) because of the additional *Hind*III restriction site present in pG). B indicates a restriction analysis (*Sac*I) of retransformation experiments for all shuttle constructs (o: original plasmid prepared from *E. coli,* r: retransformed plasmid). C indicates a restriction analysis (*Sac*I) of shuttle vectors isolated directly from *S. acidocaldarius* (o: plasmid from *E. coli,* S: plasmid from *sulfolobus* (app. 40 times more concentrated than from *E. coli*), r: retransformaned plasmid). D shows a Southern blot like in A for controls and cultures transformed with pC and pE after 200 generations of consecutive cultivation. E shows colony hybridizations for cultures from D with pRN1 specific probes.

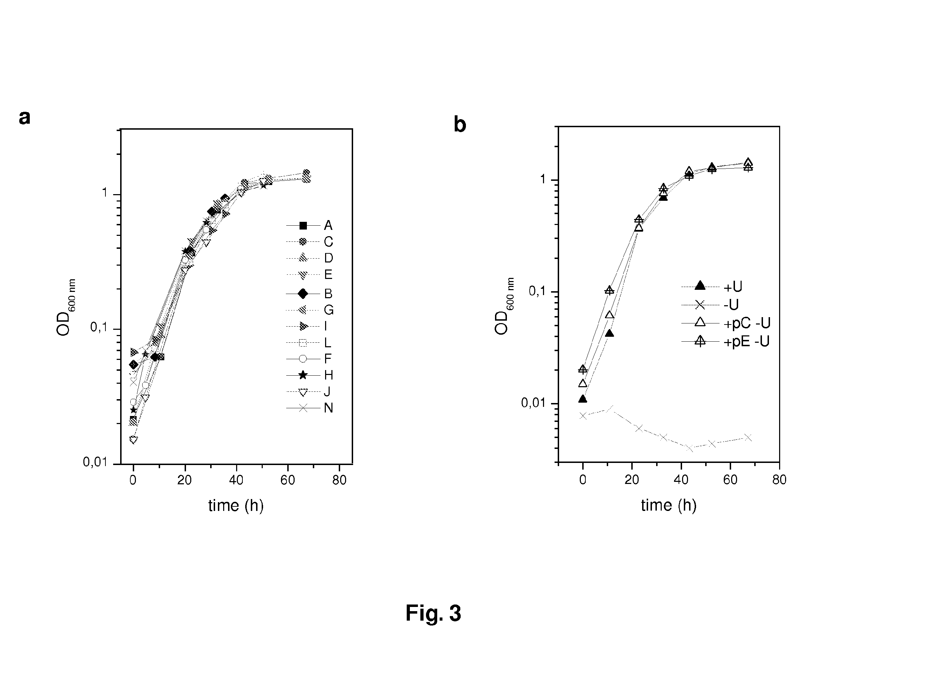

Fig. 3 depicts growth of transformants. A shows growth curves for MR31 transformed with pA to pN. B shows growth curves for the recipient strain MR31 without addition of uracil (U), with the addition of uracil and transformed with pC and pE.

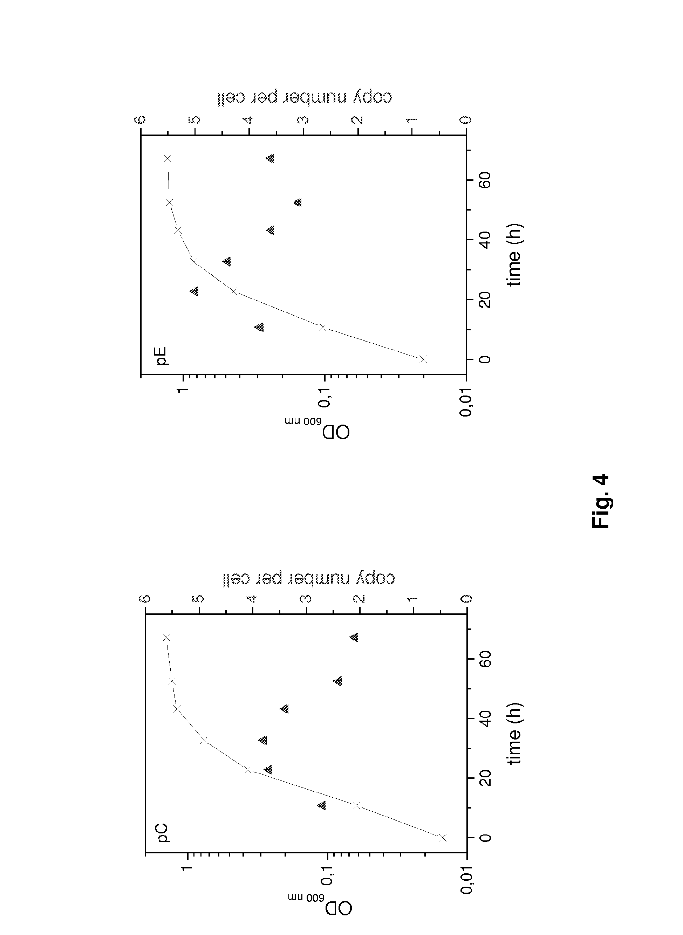

Fig. 4 shows the plasmid copy number per cell (triangles) for MR31 transformed with pC (left panel) and pE (right panel) and corresponding growth curves (line).

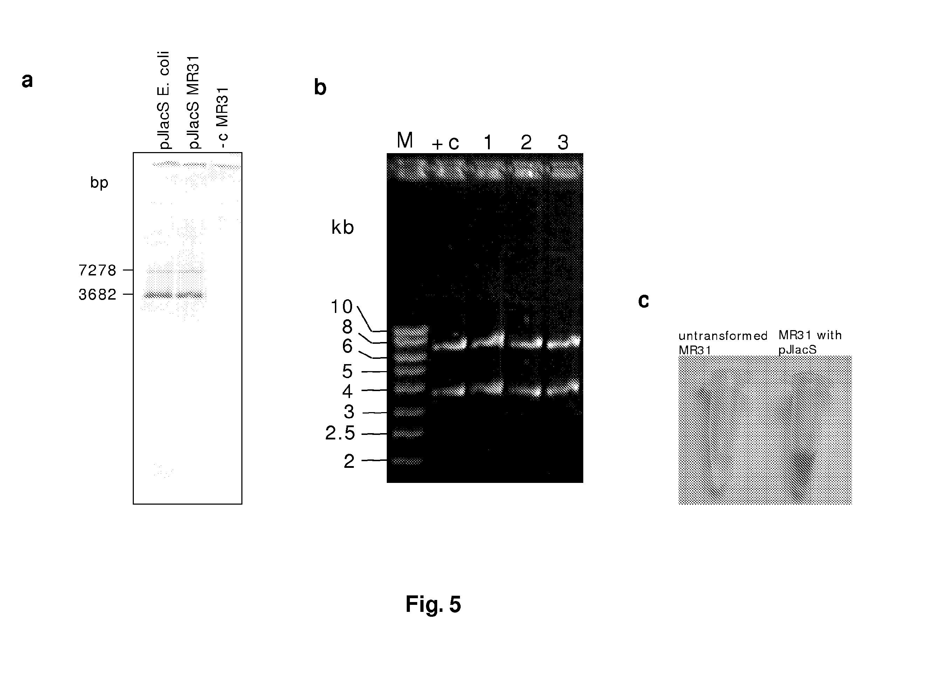

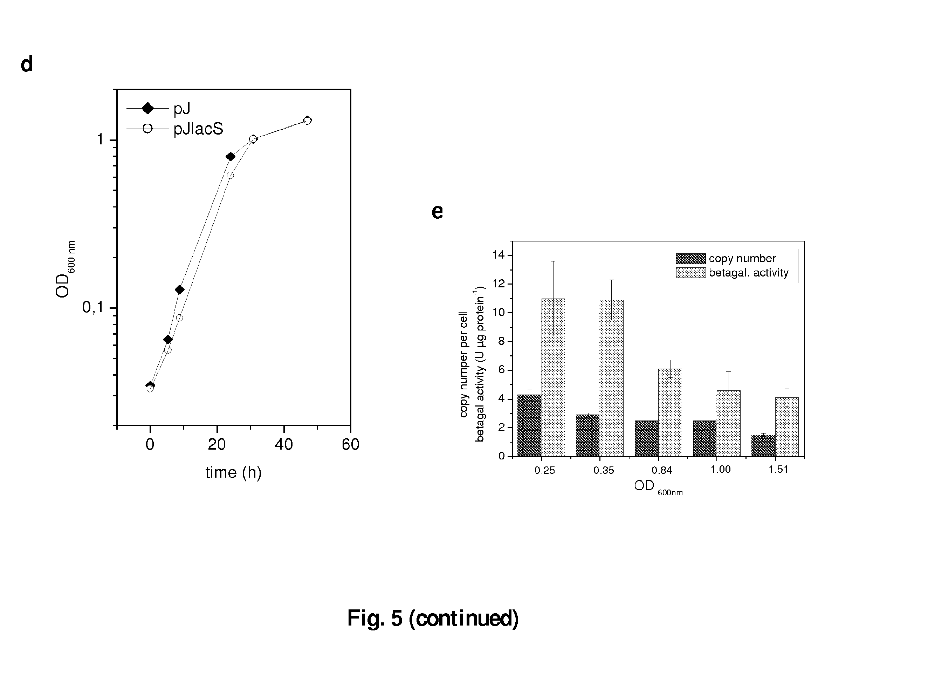

Fig. 5 indicates the replication of pJ*lacS* in *S. acidocaldarius.* A is a Southern blot (*Sac*I) of plasmid pJ*lacS* from *E. coli,* MR31 transformed with pJ*lacS* and untransformed MR31 (-c). B shows the retransformation of pJ*lacS* (*Sac*I), +c: pJ*lacS* from *E. coli,* 1,2,3: retransformants. C indicates an X-gal test with untransformed MR31 and MR31 transformed with pJ*lacS*(10 min at 75°C). D depicts growth curves for MR31 transformed with pJ (control) and with pJ/*lacS*. E illustrates a reporter gene experiment showing copy numbers of pJ*lacS* per cell and the corresponding β-galactosidase activities.

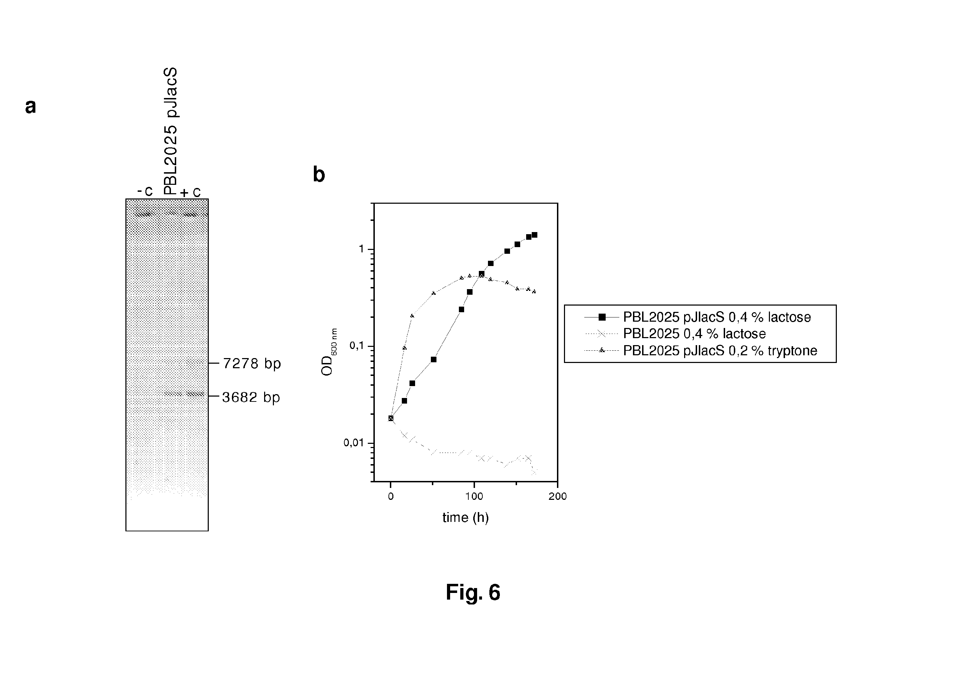

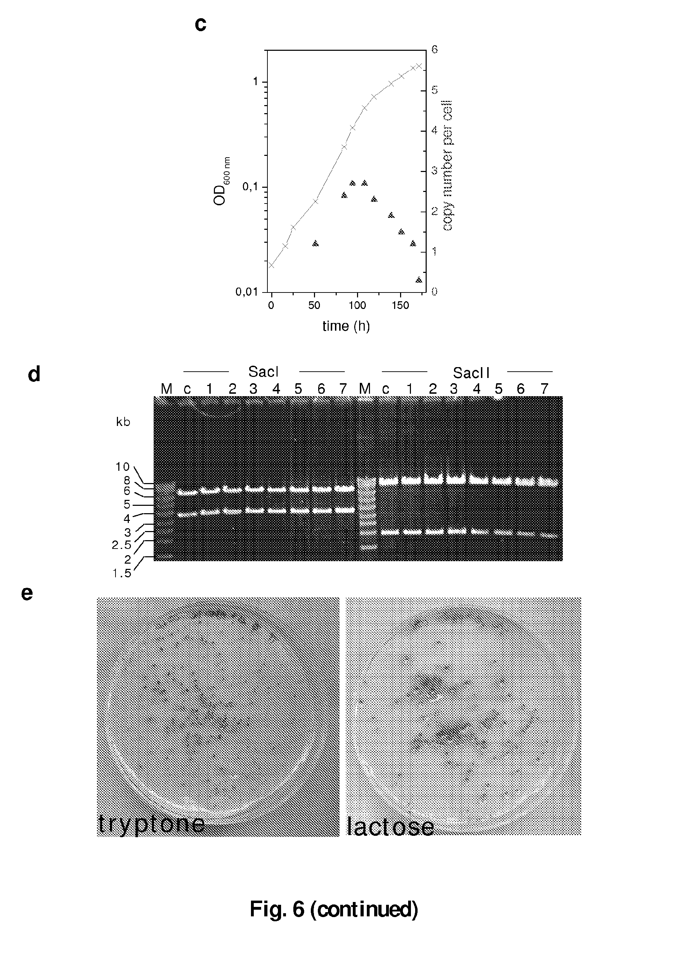

Fig. 6 indicates the replication of pJ*lacS* in *S. solfataricus.* A is a Southern blot (*Sac*I) of untransformed PBL2025 (-c), PBL2025 transformed with pJ*lacS* and pJ/*acS* from *E. coli* (+c). B depicts growth curves of untransformed PBL2025 in 0,4% lactose medium, PBL2025 transformed with pJ*lacS* in 0.4% lactose medium and in 0.2% tryptone medium. C indicates the copy numper per cell for pJ*lacS* in PBL2025 in 0.4% lactose medium. D shows the retransformation of pJ*lacS* back into *E. coli.* E indicates the plating of PBL2025 transformed with *p*J*lacS* after 100 generations of consecutive cultivation on non-selective tryptone and selective lactose plates stained with X-gal.

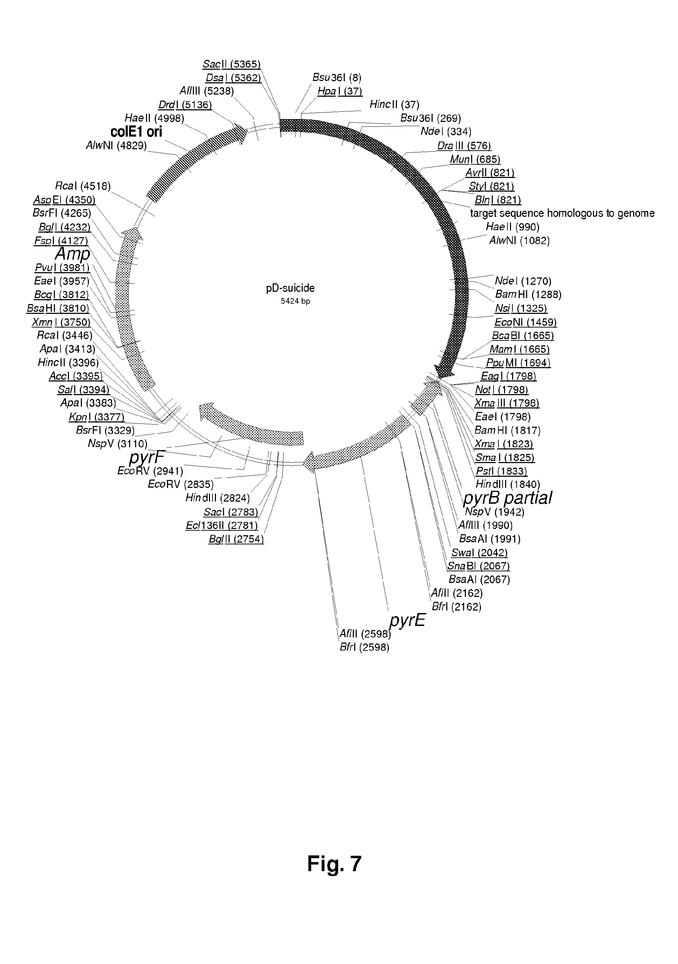

Fig. 7 shows the structure of the integrative vectors pD-Suicide. Integration into the chromosome of *S. acidocaldarius* MR31 could be demonstrated for two distinct target sequences.

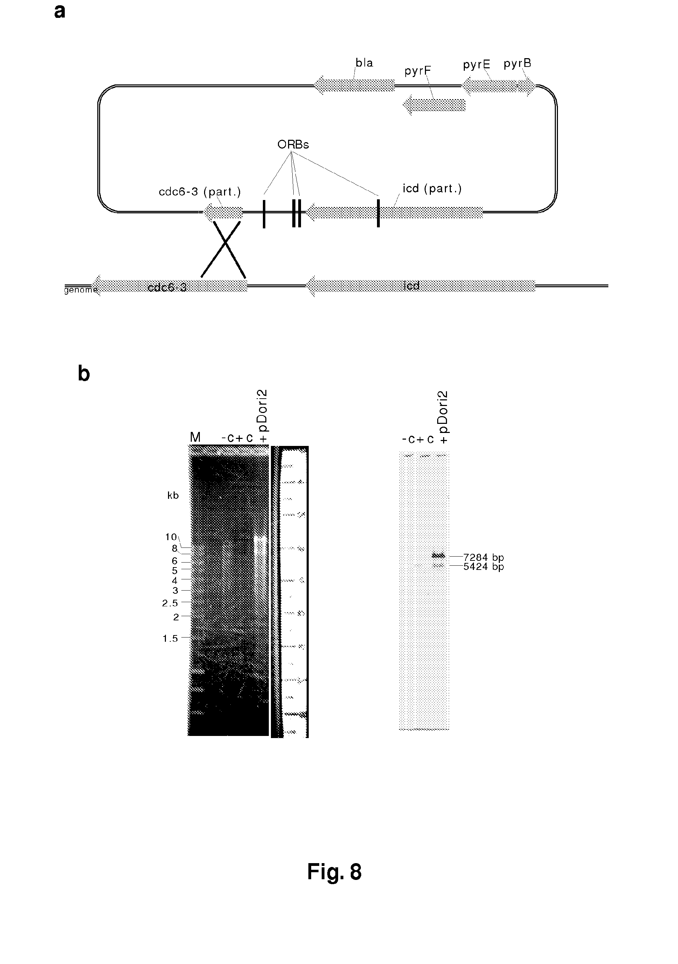

Fig. 8 demonstrates the site-specific integration of the vector pD-Suicide-ori2 into the genome of *S. acidocaldarius* MR31. A is a scheme for the location of integration sites. B is a Southern blot showing site specific single crossover integration of vector pD-Suicide-ori2: bands of the linearized non-integrated vector (5424 kb) and the integrated form of the vector (7284 kb) after digestion with PstI and hybridization to a pyrE specific probe.

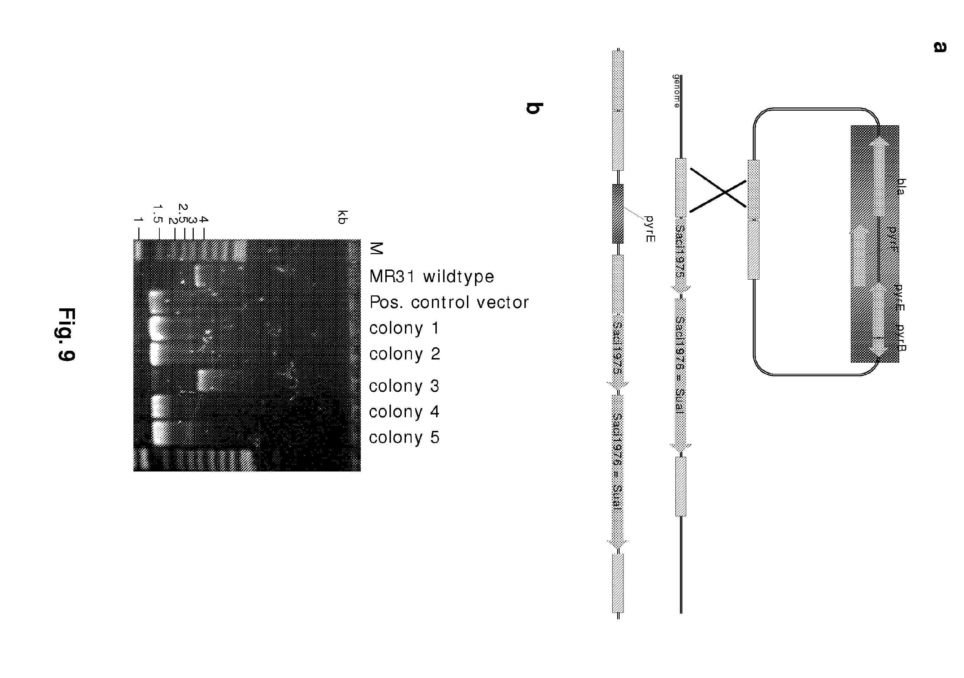

Fig. 9 demonstrates a "pop-in" method for selective modification of the *sulfolobus* genome. A is a scheme of a single crossover integration ("pop-in"). B indicates a PCR analysis of site-specific integration of the vector pD-Suicide-Sual into the genome of *S. acidocaldarius* MR31. The wildtype yields a PCR fragment of 3389 bp, whereas after integration of the vector the shorter fragment of the flanking regions alone (1276 bp) is detected. For colony 3 both bands were observed.

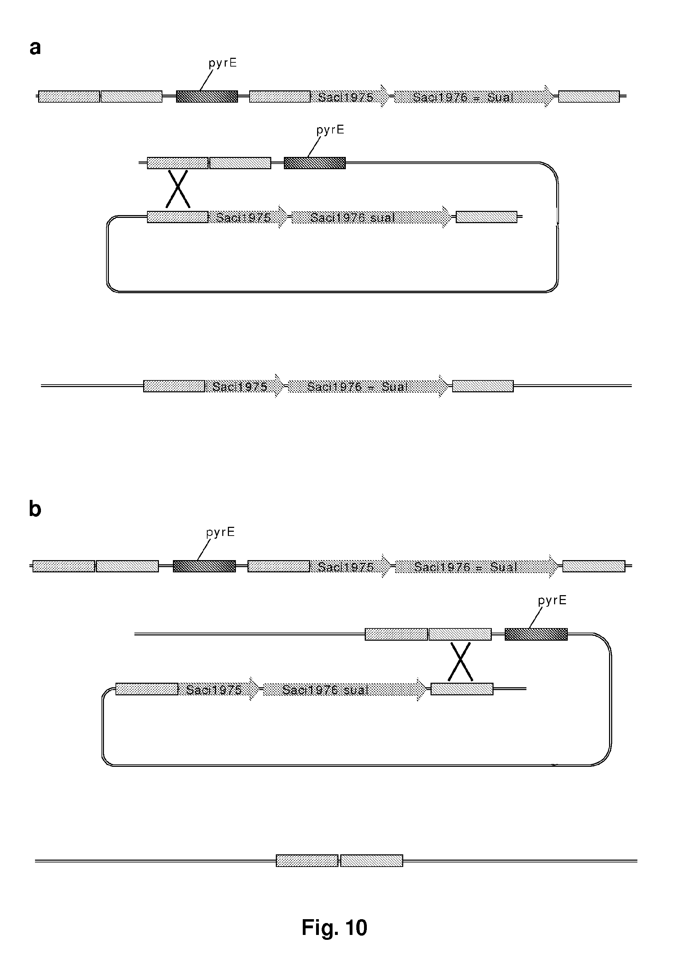

Fig. 10 demonstrates a "pop-in" method for selective modification of the *sulfolobus* genome. A and B are schemes for two possible removals of the selection marker pyrE via a second recombination from the genome (A: original sequence are restored; B: target genes knocked out: (successful pop-out))

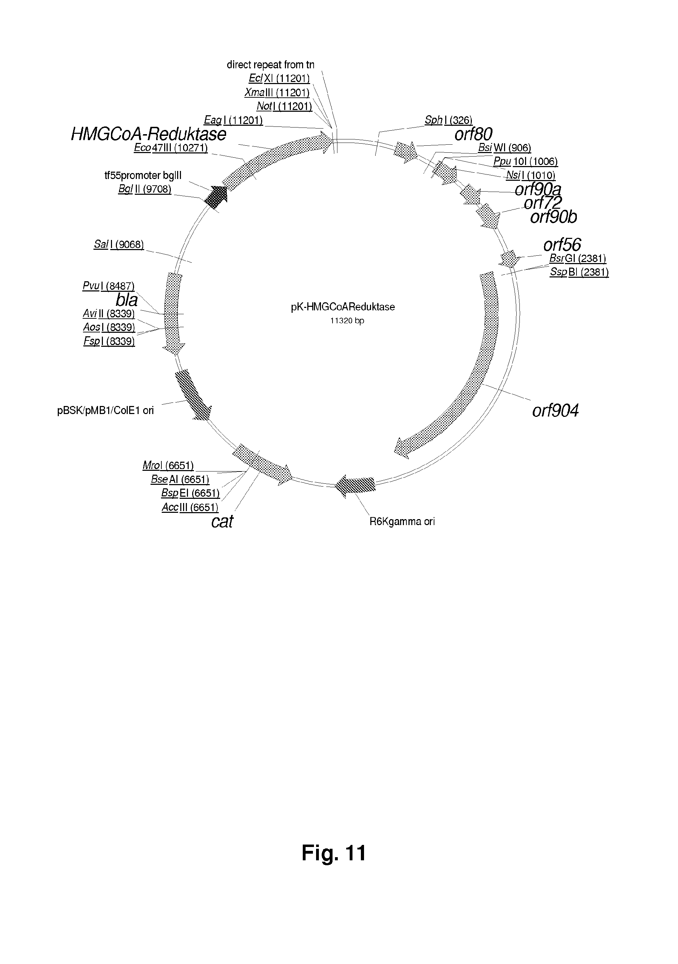

Fig. 11 illustrates the structure of the vector pK-HMGCoA-Reductase. After transformation of *Sulfolobus acidocaldarius,* transformants could be obtained by selection with the inhibitor simvastatin.

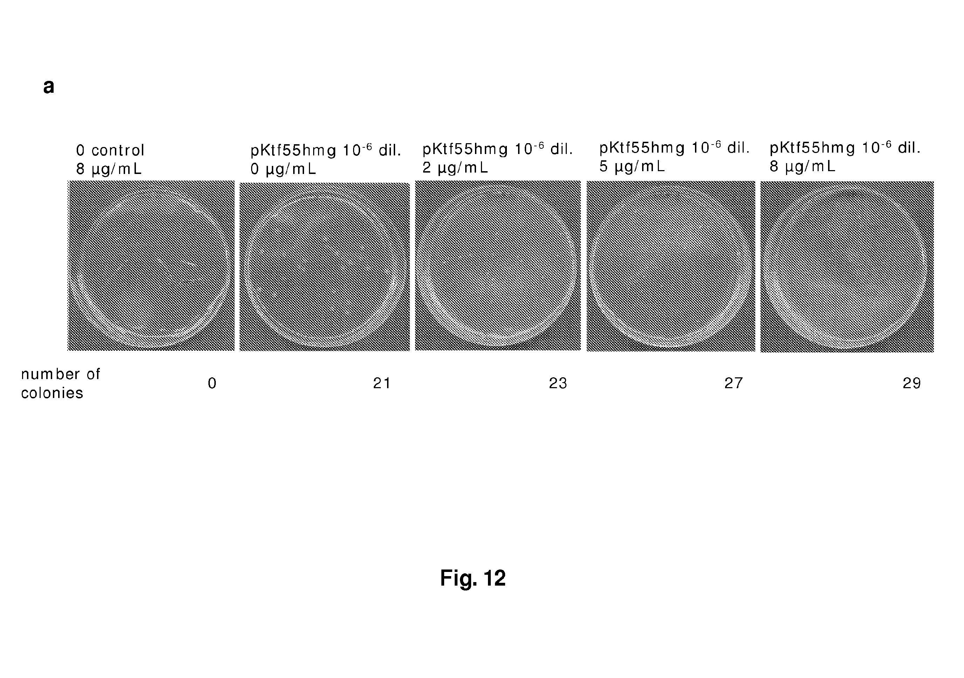

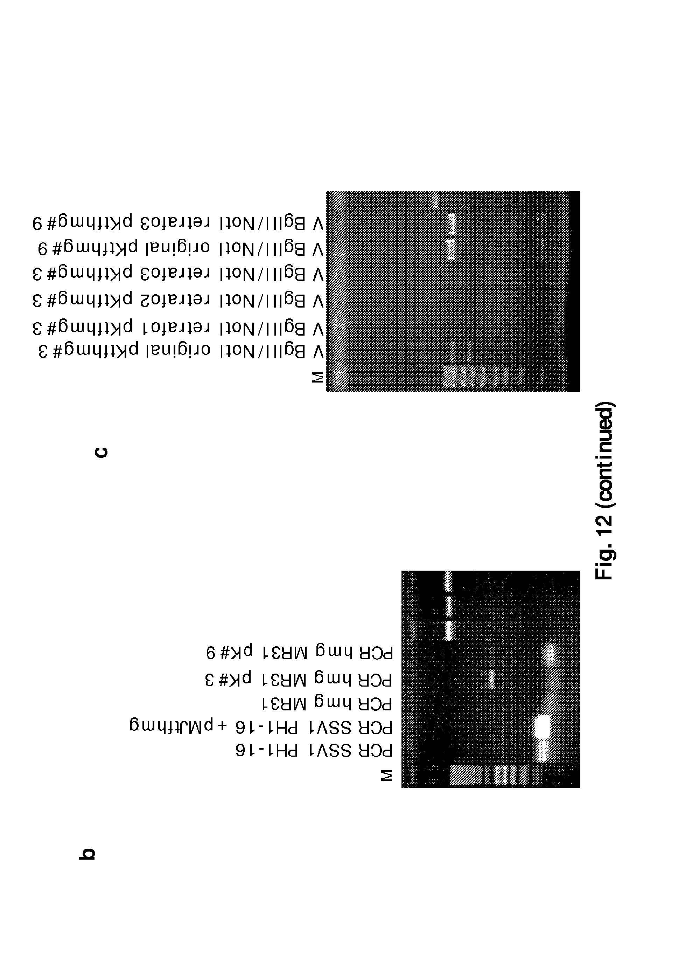

Fig. 12 demonstrates a positive selection using simvastatin. A demonstrates the selection: plating of cells after 3 rounds of preselection. Cells were transformed with pK-HMGCoA-Reductase. A control without addition of vector was included. After cultivation in medium containing 10 µg/mL simvastatin 10⁻⁶ dilutions of the control and the transformed culture were plated on simvastatin containing plates. No growth was observed for the untransformed control, whereas similar colony numbers were obtained with the transformed culture for different simvastatin concentrations. These clones were tested for plasmid presence by PCR (B) and retransformation (C). Clone #9 is correct. B shows the detection of succesful transformation of MR31 by the Hmg expression vector pK-HMGCoA-Reductase. C demonstrates the retransformation of successful vector pK-HMGCoA-Reductase into *E. coli.*

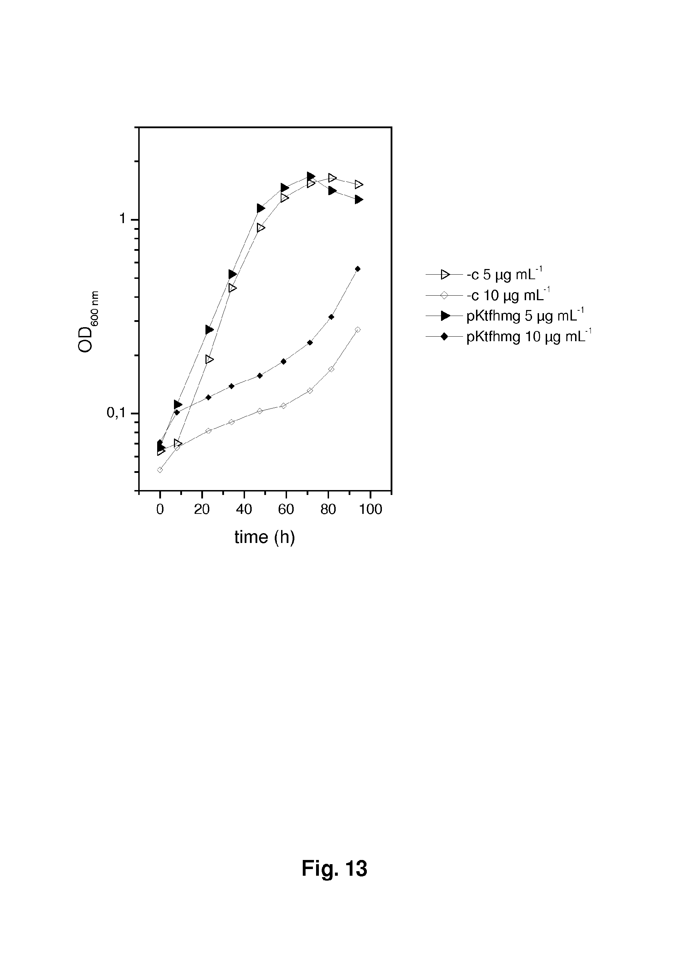

Fig. 13 shows reduced growth inhibition of cells transformed with pK-HMGCoA-Reductase (pKtfhmg). Growth curves for transformed and untransformed MR31 in the presence of 5 and 10 µg/mL simvastatin are shown.

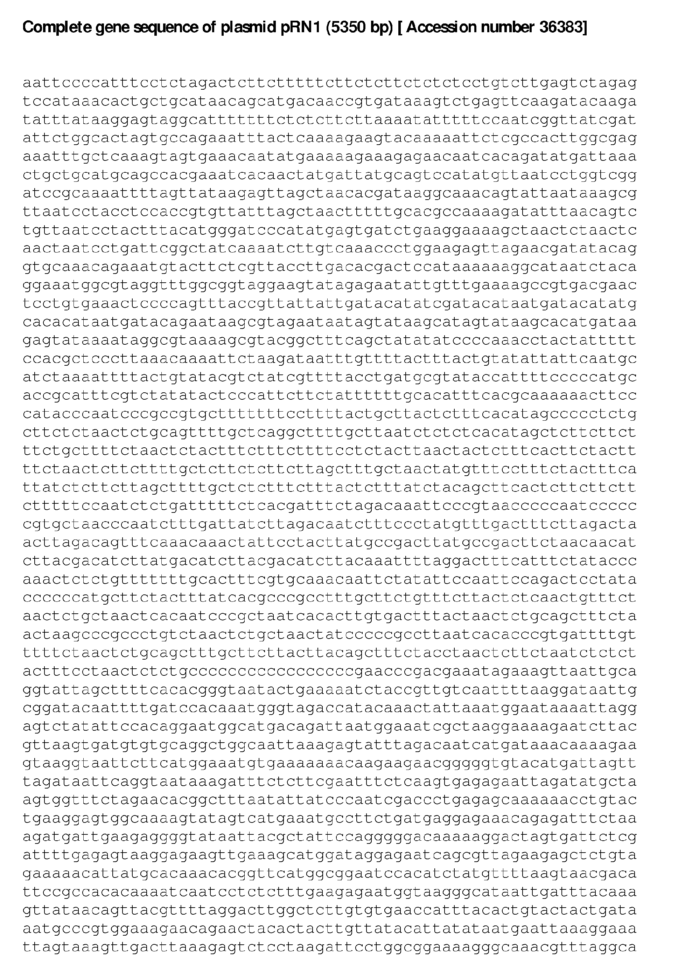

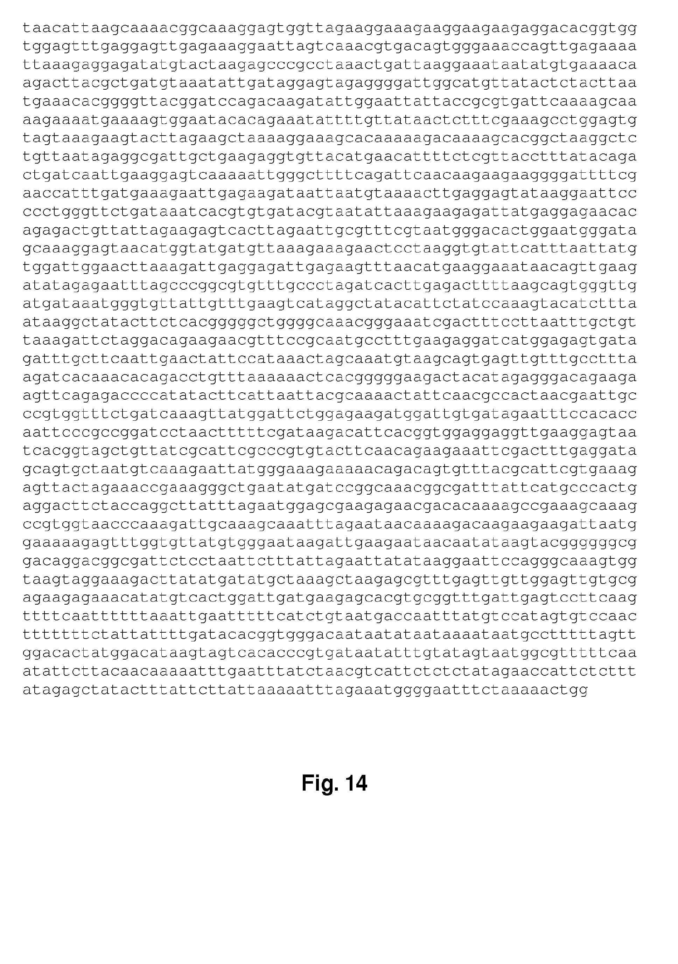

Fig. 14 shows the gene sequence of the pRN1 plasmid of *Sulfolobus islandicus.*

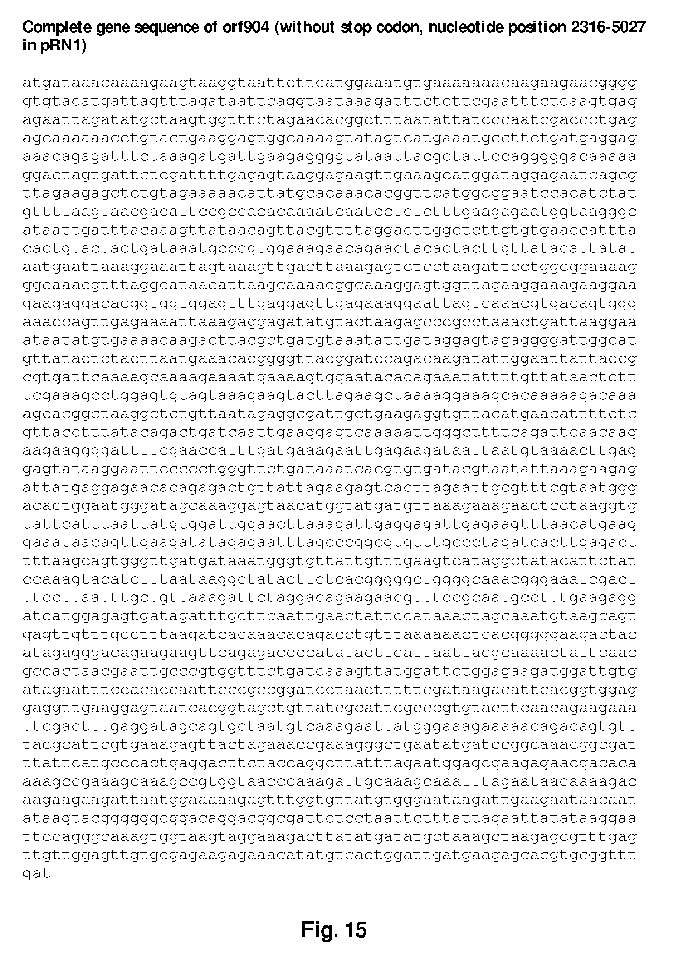

Fig. 15 shows the *orf904* gene sequence from the pRN1 plasmid of *Sulfolobus islandicus.*

The following examples illustrate the invention.

### Example 1: Construction of shuttle vectors

### General introduction

In principle, shuttle vectors can be constructed from two plasmids that replicate in different hosts simply by fusing them at two points that preserve all the important functions of each plasmid. However, in the case of pRN1, it was not clear which ORFs or intergenic regions may be important for successful replication in *sulfolobus* hosts. Therefore, transposition to generate pRN1 constructs interrupted at a number of different sites without regard to the location or its sequence context were used. From the initial transposition mixture, 13 distinct insertion points were chosen for further development, which included addition of the *pyrEF* genes of *S. solfataricus* as selectable marker (Fig. 1, Table 1).

**Table 1: Insertion sites of the transposon in constructs pA-pN. Direction of the pyrEF genes (+ same direction as orf56/orf904) as well as two unique adjacent restriction sites are given.**

| plasmid | direction of insertion of adjacent unique | | |
|---|---|---|---|
| | *pyrEF* genes | transposon (position in pRN1) | restiction sites for cloning of inserts |
| pA | + | 1693\|1694 | *Not*I*lSac*II |
| pB | - | 1940\|1939 | *Not*I/*Xma*I |
| pC | + | 1778\|1779 | *Not*I/*Sac*II |
| pD | + | 1758\|1759 | *Not*I/*Xma*I |
| pE | + | 1098\|1099 | *Not*I*lSac*II |
| pF | - | 5284\|5283 | *Not*I/*Sac*II |
| pG | + | 5309\|5310 | *Not*I*lSac*II |
| pH | + | 621\|622 | *Not*I*lSac*II |
| pI | - | 5290\|5289 | *Not*I/*Xma*I |
| pJ | + | 12\|13 | *Not*I/*Sac*II |
| pL | - | 1573\|1572 | *Not*I/*Xma*I |
| pM | + | 2334\|2335 | *Not*I/*Sac*II |
| pN | + | 355\|356 | *Not*I/*Sac*II |

In addition to providing more chances for a successful construct, this unbiased approach allowed us to evaluate possible differences in the performance of the vector constructs in *sulfolobus.* This would provide some of the first functional data regarding which of the conserved open reading frames are important for plasmid replication and maintenance.

Since the replication operon *orf56*/*orf904* was expected to be essential, only one construct interrupted within this region was chosen for analysis. The other constructs were chosen to have the interruption sites distributed as evenly as possible over the remaining part of pRN1. The open reading frames *orf80, orf90a, orf72* and *orf90b* are also interrupted in at least one construct. It was already shown that *orf90a, orf72* and *orf90b* are very unlikely to play a role in plasmid replication or maintenance in view of their very low levels of expression (36).

### Strains and growth conditions

This study used *sulfolobus solfataricus* strains PH1-16 (26) and PBL2025 (27), *sulfolobus islandicus* strains REN1 H1 (28) R1, R20, S1, R1 S1 and HVE10/4 H1 (this work), and *sulfolobus acidocaldarius* MR31 (29). Liquid cultures were grown in Brock's basal salts medium at pH 3.5 (30) or the mineral base of Grogan & Gunsalus (31), supplemented with different carbon and nitrogen sources as indicated. Acid-hydolyzed casein, i.e., NZAmine AS (Sigma), or enzymatically hydrolyzed casein, i.e., tryptone (BD Biosciences), were added at 0.1%. D-(+)-xylose was added at 0.2%, and D-(+)-lactose in "lactose-only" medium at 0.4%. For growth of untransformed *pyrEF* mutant strains PH1-16, R20, S1R1, R1, H1, and MR31, 20 µg mL⁻¹ of uracil was added to the medium. Plates were solidified by addition of 0.6 % Gelrite (Sigma) and 10 mM CaCl₂. Plates and shake flask cultures were incubated at 75°C.

### Random insertion into pRN1

The Tn5-derived transposon TnPA21 (32) was amplified by PCR using a primer (5'-CTGTCTCTTATACACATCT) complementary to the mosaic end sequence, the terminal inverted repeat sequence found on both ends of the transposon. Native pRN1 (accession number NC 001771) was isolated from a culture of *S. islandicus* REN1H1 containing only the pRN1 plasmid (33) using the Nucleo Spin plasmid extraction Kit (Macherey Nagel). Plasmid preparations from approximately 100 mL of culture were combined and ethanol-precipitated to obtain 0.1 µg of pRN1 for the transposition reaction. The transposition reaction was carried out *in vitro* using the EZ-Tn5 transposase (Epicentre) according to the instructions of the manufacturer. Transposon and plasmid were mixed at a molar ratio of 1:1. The transposition reaction products were transformed into *E. coli* EC1 00 *pir⁺* (Epicentre). The resulting transformants were screened for correctly inserted transposons by restriction digestion with *Sac*I and *Not*I. 20% of the screened colonies (a total of 80 plasmids) showed two restriction bands with a combined length of 7.2 kb and were kept for further analysis.

### Construction of pA-pN and pjlacS

Thirteen of the 80 constructs were chosen after more precise mapping of the insertion sites by restriction digestion. From these, the *E. coli* replicon (R6Kγ origin of replication and *cat* gene) introduced by TnPA21 was excised using the No*t*I and *Psp*OMI sites present on the ends of the transposon sequence. The resulting pRN1 fragments interrupted at different sites were then cloned into the No*t*I linearized vector delta2pyrEF. The plasmid delta2pyrEF is a derivative of pBluescript with the *lacZ* and f1 origin regions deleted. Specifically, pBluescriptSKII(+) was cut with *Ssp*I and *Kpn*I*,* religated, cut with *Sac*I and *Sap*I and religated. The *pyrEF* genes from *S. solfataricus* P2 were cloned into the *Sal*I and *Pst*I sites. For constructs pA to pN the transitional region from the delta2pyrEF part to the pRN1 part was sequenced to determine the exact insertion site and the direction of the transposon insertion and the direction of the cloning into the *Not*I site of delta2pyrEF. In construct pG by restriction analysis using *Hind*III an additional *Hind*III site was found to be present and confirmed by sequencing. As the pRN1 part was not PCR-amplified, but stems from the native plasmid, we conclude that pRN1 had this mutation already when isolated, or that this mutation was introduced in *E. coli* during propagation of pG. Except for this point mutation, pG corresponds to the expected sequence.

### Example 2: Transformation

### General introduction

The plasmids pA - pN were electroporated into *Sulfolobus* strains representing different species. As the *S. solfataricus pyrEF* genes provide the selectable marker, stable uracil auxotrophs were needed as recipient strains. Table 2 gives an overview of the *Sulfolobus pyrEF* mutants tested as recipients for the various pRN1 constructs.

**Table 2: Sulfolobus species and strains tested as recipient strains for the shuttle constructs pA-pN.**

| | | | | | | |
|---|---|---|---|---|---|---|
| *Sulfolobus* species | mutant and name | gene | type of mutation | | reference | successful with strain |
| S. acidocaldarius | MR31 | pyrE | deletion | 18 bp deleted | (29) | pA-pN, pJ/*acS* |
| S. islandicus REN1H1 | R1 | pyrE | point mutations | | Berkner and Lipps, unpublished | |
| S. islandicus REN1H1 | R20 | pyrEF | insertion sequence | SMN1 in promoter region | (43) | |
| S. islandicus REN1H1 | S1 R1 | pyrEF lacS | point mutations | | Berkner and Lipps, | |
| S. islandicus HVE10/4 | H1 | pyrEF | frame shift point mutations | | unpublished Berkner and Lipps, unpublished (26) (40) | |
| S. solfataricus | PH1-16 | pyrF | insertion | ISC1359 | | |
| P1 | | lacS | sequences | ISC1217 | | |
| S. solfataricus 98/2 | PBL2025 | lacS | deletion | SSO300 4-SSO305 0 deleted | | pJlacS |

For *S. solfataricus* PH1-16 and the different *S*. *islandicus* mutants, the vectors seemed to be unstable, as only very low amounts of shuttle vector could be detected in some experiments. Growth under selective conditions and positive PCR reactions with pRN1-specific primer pairs were observed, but positive results in Southern blots were never observed. Thus, the transformed cells seemed to lose the vector rapidly, and the continued growth observed on uracil-free medium may have been due to reversion, or recombinational conversion, of the *pyrEF* mutations to the wildtype sequence.
For *S. acidocaldarius* MR31, electroporation yielded distinct, rapidly growing colonies on uracil-deficient plates, and growth of these primary transformants was maintained in uracil free-liquid medium (tryptone/xylose or NZAmine/xylose). Sequencing of the chromosomal *pyrE* locus revealed that no reversion of the mutated *pyrE* sequence had occurred in these clones, consistent with the nature of this mutation (an 18-bp deletion). Only one shuttle construct, pM (disrupted replication operon *orf56*/*orf904),* consistently failed to yield Pyr⁺ transformants in MR31.

### Methylation of plasmids

For transformation into *S. acidocaldarius* shuttle constructs were methylated at the N4-position of the inner cytosine residues of GGCC recognition sequences to circumvent restriction by the *Sua*I restriction enzyme (34). Plasmids were methylated *in vivo* as previously described (35) by transforming the shuttle constructs into *E. coli* ER1821 (New England Biolabs) bearing the additional plasmid pM.*Esa*BC41 (New England Biolabs). Complete methylation was confirmed by the absence of any cutting after incubation with 5 U *Hae*III for 1 to 4 h.

### Electroporation

Constructs were electroporated either using a Gene Pulser I or Gene Pulser II (BioRad) following the protocol of Schleper et al. (1992) or using a Gene Pulser Xcell (BioRad) with a constant time protocol with input parameters 1500 V, 10.2 ms, 2mm cuvettes, or using the protocol described by Kurosawa and Grogan (35). For *S. acidocaldarius,* regeneration was done for 30 - 40 min in tryptone/xylose medium, water or recovery solution before plating on tryptone/xylose plates or NZAmine/xylose plates. For lactose utilization in *S. solfataricus,* plating after electroporation was not feasible. Instead, electroporated cells were directly transferred into preheated lactose medium and cultivated in 50 mL flasks.

### Retransformation

1 µL of genomic DNA preparation or 1-5 µL of plasmid prepared from *Sulfolobus* by alkaline lysis were transformed into RbCl-competent *E. coli* XL1-Blue cells or into the *mcrBC* deficient *E. coli* strain ER2267 (New England Biolabs).

### Example 3: Characterization of shuttle vectors

### Shuttle constructs are stable and do not integrate or rearrange

Using Southern blots, it was examined whether the shuttle constructs pA-pN were present in the *S. acidocaldarius* clones selected after electroporation (Fig. 2A). This analysis confirmed that the vector constructs had the correct size and did not integrate into the host genome, as no bands in addition to the expected ones for the episomal form of the vectors were observed. In addition, none of the vector constructs were observed to undergo large rearrangements in *S. acidocaldarius,* with the exception of pB. For this construct, an additional band of app. 6 kb was observed in the Southern blot, which indicated a rearrangement occurring in the *Sulfolobus* host.

Next, it was tested by retransformation experiments if the original shuttle plasmids could be recovered intact from *S. acidocaldarius* transformants. As shown in Fig. 2B, five to thirty retransformants per construct were checked by restriction analysis and only the correct restriction pattern was observed. In the case of pA, pC, pD and pE, the shuttle plasmids were also isolated directly from transformed *Sulfolobus* cultures and analyzed by restriction digestion (Fig. 2C).

From the initial set of constructs, plasmids pC and pE were chosen to evaluate long-term stability under selective conditions. Cultures of pC and pE transformants were cultivated continuously for approximately 200 generations without uracil supplementation. Then retransformation experiments and Southern blots were repeated (Fig. 2D), with the same results.

The data of Fig. 3 indicate that direction of the insertion in a given region does not influence performance of the vector. The vectors pF, pl and pG, for example, have insertion sites within 15 nt of each other. In pG, the *pyrEF* genes are oriented clockwise, in pl and pF counter clockwise, without detectable effects on plasmid stability or growth (Fig. 3). In addition, the growth phenotype of transformed cells is comparable to that of the untransformed recipient strain when supplemented with uracil.

To test if the selection for uracil prototrophy ensures that every cell contains a shuttle vector, cells were plated on selective NZAmine/xylose medium and on non-selective tryptone/xylose medium supplemented with uracil. In eight different experiments comparable colony numbers were obtained on selective and non-selective plates showing that no cells escaped the selection. To prove that the vast majority of cells contained a shuttle vector, cells transformed with constructs pC and pE were plated on non-selective plates and examined by colony hybridizations with pRN1 specific probes (Fig. 2E).

### Southern blots

Genomic DNA was prepared from 1 mL of culture using the Chemagenic DNA Bacteria Kit (Chemagen, Baesweiler, Germany) according to the instructions of the manufacturer. After digestion with either *Hind*III for constructs pA-pN or *Sac*I for pJ*lacS* restriction fragments were resolved in 1% agarose gels, transferred to a Hybond N membrane (Amersham) by capillary transfer, fixed by UV irradiation for 5 min on a UV transilluminator and hybridized to digoxigenin-labeled probes complementary to the *pyrE* gene (position 9-320 from the start of the *pyrE* gene from *S. solfataricus* P2) and pRN1 (position 4892-5048 in pRN1) for pA to pN or *lacS* (position 1124-1438 from the start of the *lacS* gene from *S. islandicus* REN1H1) and pRN1 for pJ*lacS.* Labeling and detection was done using the PCR DIG Probe Synthesis Kit and the Digoxigenin Labeling and Detection Kit (Roche).

### Colony hybridization

Colonies from plates were transferred to Hybond N membranes and subsequently incubated for 10 min on a filter paper soaked with 0.5 M NaOH, 1.5 M NaCl then for 10 min on a filter paper soaked with 1 M Tris-HCl (pH 7,5), 1.5 M NaCl, then for 5 min on a filter paper soaked with 10x SSC. Membranes were cross-linked for 5 min on the 10x SSC filter paper using a transilluminator. Hybridization and detection were done as described for Southern blots with pRN1-specific probes.

### Plasmid copy number determination

Copy numbers of the different shuttle constructs were determined as already described (36) by qPCR and cell number determination through plating, respectively.

### Vector retention and copy number

The facile generation of Pyr⁺ colonies by electroporation and direct plating on selective medium indicated that all the constructs tested, except for construct pM, could replicate in *S. acidocaldarius* under appropriate selection. In order to provide a more stringent and quantitative comparison of these constructs, their retention in populations growing in non-selective, uracil-supplemented liquid medium was monitored. Specifically, the fraction of Pyr⁺ cells in the population at three different times was determined, by dilution and plating on uracil-supplemented and unsupplemented plates.

Fig. 3C shows the retention of 13 constructs over approximately 10 generations, corresponding to about 1000-fold numerical expansions of the host cell populations. Most constructs showed measurable loss under these conditions, resulting in about 10% Pyr⁺ cells in the cultures. In a few cases, however, plasmid retention was much lower. The most severe instability was seen in construct pH, in which the *orf80* gene is interrupted. This result provided evidence that the small DNA-binding protein encoded by *orf80* has an important role in the stable replication of pRN1 and related plasmids. Intermediate instability was observed for construct pJ*lacS* (described below). It was suspected that this construct with the very strong *tf55α* promoter is a burden for the cell. Both pJ*lacS* and pH yielded small or heterogeneous colonies when streaked on selective plates, consistent with the observed instability under non-selective conditions.

According to qPCR results, all constructs showed copy numbers within the range of 2-8 copies per cell, except for pB that showed low copy numbers around 1. For pC and pE, the time course of the copy number during batch fermentation was also determined (Fig. 4). The copy number increased in early and mid-log phase and decreased in stationary/death phase. This behavior has also been observed for the wild type pRN1 plasmid (36). The copy number of the wild type plasmid in its original host strain grown on rich media containing yeast extract is higher, reaching 20 copies per cell. The difference might be due to differences in medium composition, because the use of yeast extract in the medium for the shuttle constructs is not feasible as it contains pyrimidines.

### Suitability for protein expression or reportergene tests

To test whether the vector tolerates the insertion of sequences containing expressed *sulfolobus* genes, the *tf55αlacS* expression cassette (20) was cloned into the *Sac*II restriction site of pJ generating the vector pJ*lacS.* The stability of the construct was tested by retransformation into *E. coli* and Southern blotting (Fig. 5A & B). The construct turned out to be stably replicated in *S. acidocaldarius.* Staining with 5-bromo-4-chloro-3-indolyl-β-D-galactoside (X-gal) revealed that the β-glycosidase was expressed under the control of the heat shock promoter (Fig. 5C). This test could be done without prior isolation of a *lacS* mutant of strain MR31 because the endogenous β-glycosidase activity is very low in *S. acidocaldarius.* The enzyme activity was also measured quantitatively (as β-galactosidase) at different ODs of a MR31 culture transformed with *pJlacS.* Copy numbers were determined simultaneously and it was found that measured β-galactosidase activities correlated well with vector copy numbers (Fig. 5E), as previously observed (16;20). It should be noted that the β-galactosidase (2-11 U/mg) is much higher than the wild type β-galactosidase activity of *S. solfataricus* (0.2 U/mg) (36) and is comparable to the β-galactosidase activity in a viral overexpression system (1.5 -5 U/mg) (20).

### β-galactosidase assay

For convenience, the broad-specificity β-D-glycosidase (37;38) encoded by the *S. solfataricus lacS* gene was assayed as β-galactosidase activity. Crude extracts were prepared by a freeze-thaw method (20) in which cells were resuspended in 50 mM Na-phosphate buffer, pH 7, and subjected to 5 freeze-thaw cycles (-196°C/+50°C). After centrifugation for 30 min at 10000 rpm the supernatant was stored at -20°C, or assayed directly. All β-galactosidase assays were conducted in triplicate in a 75°C bench top shaker. The reaction mixture consisted of 1 µL of crude extract (or water for blanks), 92 µL of 50 mM Na-phosphate buffer, pH 7 and the assay was started by addition of 7 µL of 12 mg mL⁻¹ ortho-nitrophenyl-β-D-galactopyranoside (ONPG) solution. Incubation was continued for 5 min before the tubes were rapidly cooled on ice and 100 µL of 1 M Na₂CO₃ solution was added to stop the reaction. Concentration of ONP was subsequently determined in a 96-well plate in a plate reader at 410 nm using a standard curve generated with ONP. Protein concentration of the crude extracts was determined by the method of Ehresmann (39).

### Replication in S. solfataricus

Demonstration of *lacS* expression in *S. acidocaldarius* pJ*lacS* transformants suggested the possibility of a selection by conferring, or restoring, the ability to catabolize lactose or other β-glycosides. The *S*. *solfataricus* 98/2 *lacS* deletion mutant PBL2025 (40) was therefore tested for complementation by plasmid pJ*lacS.* After three rounds of selection in liquid medium (0.4% lactose), >95% of all cells contained a shuttle vector, as shown by plating on selective lactose versus non-selective tryptone plates, and X-gal staining of colonies. On both plates equal numbers of colonies were observed, on the non-selective plate in addition to approximately 300 colonies also seven white colonies were observed (Fig. 6). Southern blot, retransformation, growth and copy number determinations for pJ*lacS* in *S. solfataricus* are summarized in supplementary Fig. 6 and indicate that pJl*acS* is stably replicated in *S. solfataricus.*

### X-gal staining

A qualitative β-galactosidase assay was based on hydrolysis of 5-bromo-4-chloro-3-indolyl-β-D-galactoside (X-gal). For liquid cultures 200 µL of culture were mixed with 20 µL of substrate solution (20 mg/mL in dimethylformamide) and incubated at 75°C until color development was observed. To score colonies, plates were sprayed with the same X-gal solution and incubated at 75°C.

### Transformation efficiencies

For *S*. *acidocaldarius* direct determination of transformation efficiencies is possible, because plating of the primary electroporation mixture can be done after only 30 min of regeneration. Considering all transformations performed in the current study (n = 150), the efficiencies range from 1*10² to 6*10⁴ transformants per microgram plasmid DNA. The composition of the selective plates had an influence. Low efficiencies of around 10² transformants per µg of DNA were obtained when using very selective NZAmine/xylose plates, whereas higher efficiencies were obtained with tryptone/xylose plates. The batch of electrocompetent cells, electroporation protocol and regeneration procedure also had an influence, as already described (35).

On tryptone/xylose plates, the formation of very small colonies - that did not contain a shuttle vector - was observed in addition to the colonies of normal size that were able to grow in selective liquid medium. Controls without electroporation or without addition of shuttle vector also yielded small colonies, which were not able to grow when restreaked on selective plates or cultivated in liquid medium. Based on their phenotype and frequency in strain MR31, it was hypothesize that these "pseudo-transformants" contain spontaneous mutations elsewhere in the *S. acidocaldarius* chromosome that partially suppress the *pyrE* phenotype.

Finally, it was confirmed that complete methylation of the shuttle vectors is essential for efficient transformation of *S. acidocaldarius.* None of the constructs pA-pN yielded transformants when unmethylated or partly protected plasmids were electroporated.

### Summary

Based on the results with various constructs and recipient strains, it is concluded that the primary obstacle to establishing stably replicating shuttle vectors derived from *sulfolobus* plasmid pRN1 is not preservation of critical plasmid functions or identification of a required host species, but creation of a suitably reliable selection. For example, point and transposon mutants of *S. islandicus* or *S. solfataricus* that showed low reversion frequencies in small scale fluctuation tests (15*10⁻⁹ - < 6*10⁻⁹ reversions per cell division, unpublished results) displayed for unknown reasons higher reversion frequencies after electroporation with a shuttle construct. These problems could be avoided by the use of a *pyrE* deletion mutant of *S. acidocaldarius.* In contrast to *S. solfataricus* and *S. islandicus,* background growth on selective plates was not observed with *S. acidocaldarius.* Although the basis of this difference has not been established, *S. acidocaldarius* lacks homologues of the cytosine/uracil/thiamine/allantoin permeases (SSO1905, SSO2042) present in *S. solfataricus* and *S. tokodaii* (ST1564) that might facilitate growth on medium with very low uracil concentrations.

Under selective conditions the vectors could be faithfully propagated in *Sulfolobus.* Rearrangements occurred in only two cases, pB and pJ*lacS,* and only after many generations. We do not know why pB behaves differently in this respect, although it is the only construct interrupted in between *orf90b* and *orf56.* This region of pRN1 contains several repeats and other remarkable features like a stretch of 17 consecutive C residues (41). An interruption in this region is obviously not as well tolerated as in other regions. The only interruption site that abolished shuttle vector replication completely was that of construct pM, and is situated within the cotranscribed replication operon *orf56*/*orf904.* For pM no viable transformants could be isolated. The other conserved open reading frame, *orf80,* also called *p*/*rA* (plasmid regulatory), that is present on almost all sequenced genetic elements of *sulfolobus* (42) is interrupted in pH. Interestingly, pH shows growth comparable to the other constructs and yields the same transformation efficiencies. Therefore *orf80* seems not to be essential for replication and maintenance of pRN1, at least not when selective pressure is applied. However, under non-selective conditions, construct pH was lost at a much faster rate than any other construct that could be successfully established in *S. acidocaldarius.* The relative instability of this construct provides the first experimental evidence that the DNA-binding protein ORF80 has an important role in stable replication of pRN1. The instability of this construct may also have practical uses. For example, it may facilitate transfer of *pyrEF*-marked genes to the host chromosome, by allowing such genes to be first established on an episome, and then stabilized in the population by recombinational integration at the homologous locus.

Many shuttle constructs developed so far for hyperthermophilic archaea have been observed to rearrange in *E. coli* (18), which hampers the use of these systems. Some of these problems may, in principle, be circumvented by the use of *E. coli* strains designed specially for dealing with unstable constructs. Additionally reducing the growth temperature to 30°C and using only 50 µg mL⁻¹ of ampicillin is necessary to prevent rearrangements in the pMJ vector system (22). Rearrangements for the construct pJ*lacS* in one out of 10 preparations of this plasmid in *E. coli* XL1-Blue cells at 37°C and 100 µg mL⁻¹ of ampicillin were observed. In general, however, the constructs pA to pN seemed to be fully stable in *E. coli.* In particular, rearrangements in retransformed plasmids were never detected. In Southern blots, faint traces of plasmid rearrangements were visible for some preparations from *E. coli* but did not interfere with successful transformation of *Sulfolobus.*

As a result, multicopy, non-integrative, plasmid-based *Sulfolobus-E. coli* shuttle vectors that are very stable in both hosts, and are suitable for the use, e.g. in protein expression and reporter gene studies were developed. Transformation is rapid and simple, involving electroporation of stable *pyrE* mutants and plating on uracil-deficient media. The constructs are small, enabling direct cloning into unique *Sac*II/*Xma*I and *Not*I restriction sites. The host range so far comprises *S. acidocaldarius* and *S. solfataricus,* the two most widely used and best studied species of *Sulfolobus* for which genome sequence information is available. The presence of the shuttle constructs in the cells does not cause significant growth retardation and there is limited risk of accidentally contaminating cultures because the vectors are not infectious. It should be emphasized that performance of these shuttle constructs has now been confirmed independently in three different laboratories using slightly different electroporation and cultivation protocols.

In addition, use of *S. acidocaldarius* as a recipient strain has certain practical advantages which mitigate somewhat the inconvenience of requiring specific DNA methylation. *S. acidocaldarius* does not contain any integrated copies of pRN1 or genes homologous to pRN1 genes. This enables detailed experiments on essential regions and proteins for pRN1 replication and maintenance without interference from plasmid-gene homologues located on the host chromosome. Because of the low sequence similarity between *S*. *acidocaldarius* and *S. solfataricus* there is also minimal risk of undesired homologous recombination when cloning genes of *S*. *solfataricus* into the shuttle vector, e.g. for protein expression. *S. acidocaldarius* is the only *sulfolobus* species so far that does not contain active insertion sequences and seems to be genetically stable (12). In addition it is the *Sulfolobus* species showing the highest growth rate with doubling times of around 3-4 hours during exponential growth, and exhibits efficient homologous recombination (35). In this context, the series of pRN1 shuttle vectors which were constructed promises to add detailed genetic analyses to the already advanced biochemical characterization of various *Sulfolobus* gene products.

### References

1. Agback,P., Baumann,H., Knapp,S., Ladenstein,R. and Hard,T. (1998) Architecture of nonspecific protein-DNA interactions in the Sso7d-DNA complex. Nat.Struct.Biol., 5, 579-584.
2. Bell,S.D., Botting,C.H., Wardleworth,B.N., Jackson,S.P. and White,M.F. (2002) The interaction of Alba, a conserved archaeal chromatin protein, with Sir2 and its regulation by acetylation. Science, 296, 148-151.
3. Robinson,N.P., Dionne,l., Lundgren,M., Marsh,V.L., Bernander,R. and BeII,S.D. (2004) Identification of two origins of replication in the single chromosome of the archaeon Sulfolobus solfataricus. Cell, 116, 25-38.
4. Lundgren,M. and Bernander,R. (2007) Genome-wide transcription map of an archaeal cell cycle. Proc.Natl.Acad.Sci. U.S.A.
5. Ling,H., Boudsocq,F., Woodgate,R. and Yang,W. (2001) Crystal structure of a Y-family DNA polymerase in action: a mechanism for error-prone and lesion-bypass replication. Cell, 107, 91-102.
6. Qureshi,S.A., Bell,S.D. and Jackson,S.P. (1997) Factor requirements for transcription in the Archaeon Sulfolobus shibatae. EMBO J., 16, 2927-2936.
7. Condo,I., Ciammaruconi,A., Benelli,D., Ruggero,D. and Londei,P. (1999) Cis-acting signals controlling translational initiation in the thermophilic archaeon Sulfolobus solfataricus. Mol.Microbiol., 34, 377-384.
8. Rosendal,K.R., Wild,K., Montoya,G. and Sinning,l. (2003) Crystal structure of the complete core of archaeal signal recognition particle and implications for interdomain communication. Proc.Natl.Acad.Sci.U.S.A, 100, 14701-14706.
9. Snijders,A.P., Walther,J., Peter,S., Kinnman,l., de Vos,M.G., van de Werken,H.J., Brouns,S.J., Van der,O.J. and Wright,P.C. (2006) Reconstruction of central carbon metabolism in Sulfolobus solfataricus using a two-dimensional gel electrophoresis map, stable isotope labelling and DNA microarray analysis. Proteomics., 6, 1518-1529.
10. She,Q., Singh,R.K., Confalonieri,F., Zivanovic,Y., Allard,G., Awayez,M.J., Chan-Weiher,C.C., Clausen,I.G., Curtis,B.A., De Moors,A. et al. (2001) The complete genome of the crenarchaeon Sulfolobus solfataricus P2. Proc.Natl.Acad.Sci.U.S.A, 98, 7835-7840.
11. Kawarabayasi,Y., Hino,Y., Horikawa,H., Jin-no,K., Takahashi,M., Sekine,M., Baba,S., Ankai,A., Kosugi,H., Hosoyama,A. et al. (2001) Complete genome sequence of an aerobic thermoacidophilic crenarchaeon, sulfolobus tokodaii strain7. DNA Res., 8, 123-140.
12. Chen,L., Brugger,K., Skovgaard,M., Redder,P., She,Q., Torarinsson,E., Greve,B., Awayez,M., Zibat,A., Klenk,H.P. et al. (2005) The genome of sulfolobus acidocaldarius, a model organism of the Crenarchaeota. J,Bacteriol., 187, 4992-4999.
13. Snijders,A.P., Walther,J., Peter,S., Kinnman,l., de Vos,M.G., van de Werken,H.J., Brouns,S.J., Van der,O.J. and Wright,P.C. (2006) Reconstruction of central carbon metabolism in Sulfolobus solfataricus using a two-dimensional gel electrophoresis map, stable isotope labelling and DNA microarray analysis. Proteomics*..*
14. Barry,R.C., Young,M.J., Stedman,K.M. and Dratz,E.A. (2006) Proteomic mapping of the hyperthermophilic and acidophilic archaeon sulfolobus solfataricus P2. Electrophoresis*.*
15. Allers,T. and Mevarech,M. (2005) Archaeal genetics - the third way. Nat.Rev.Genet., 6, 58-73.
16. Aucelli,T., Contursi,P., Girfoglio,M., Rossi,M. and Cannio,R. (2006) A spreadable, non-integrative and high copy number shuttle vector for sulfolobus solfataricus based on the genetic element pSSVx from sulfolobus islandicus. Nucleic Acids Res., 34, e114.
17. Cannio,R., Contursi,P., Rossi,M. and Bartolucci,S. (1998) An autonomously replicating transforming vector for sulfolobus solfataricus. J.Bacteriol., 180, 3237-3240.
18. Aravalli,R.N. and Garrett,R.A. (1997) Shuttle vectors for hyperthermophilic archaea. Extremophiles., 1, 183-191.
19. Contursi,P., Pisani,F.M., Grigoriev,A., Cannio,R., Bartolucci,S. and Rossi,M. (2004) Identification and autonomous replication capability of a chromosomal replication origin from the archaeon Sulfolobus solfataricus. Extremophiles., 8, 385-391.
20. Jonuscheit,M., Martusewitsch,E., Stedman,K.M. and Schleper,C. (2003) A reporter gene system for the hyperthermophilic archaeon Sulfolobus solfataricus based on a selectable and integrative shuttle vector. Mol.Microbiol., 48, 1241-1252.
21. Stedman,K.M., Schleper,C., Rumpf,E. and Zillig,W. (1999) Genetic requirements for the function of the archaeal virus SSV1 in Sulfolobus solfataricus: construction and testing of viral shuttle vectors. Genetics, 152, 1397-1405.
22. Albers,S.V., Jonuscheit,M., Dinkelaker,S., Urich,T., Kletzin,A., Tampe,R., Driessen,A.J. and Schleper,C. (2006) Production of Recombinant and Tagged Proteins in the Hyperthermophilic Archaeon Sulfolobus solfataricus. Appl.Environ.Microbiol., 72, 102-111.
23. Lipps,G., Rother,S., Hart,C. and Krauss,G. (2003) A novel type of replicative enzyme harbouring ATPase, primase and DNA polymerase activity. EMBO J., 22, 2516-2525.
24. Lipps,G., Stegert,M. and Krauss,G. (2001) Thermostable and site-specific DNA binding of the gene product ORF56 from the Sulfolobus islandicus plasmid pRN1, a putative archael plasmid copy control protein. Nucleic.Acids.Res., 29, 904-913.
25. Lipps,G., lbanez,P., Stroessenreuther,T., Hekimian,K. and Krauss,G. (2001) The protein ORF80 from the acidophilic and thermophilic archaeon sulfolobus islandicus binds highly site-specifically to double-stranded DNA and represents a novel type of basic leucine zipper protein. Nucleic Acids Res., 29, 4973-4982.
26. Martusewitsch,E., Sensen,C.W. and Schleper,C. (2000) High spontaneous mutation rate in the hyperthermophilic archaeon sulfolobus solfataricus is mediated by transposable elements. J.Bacteriol., 182, 2574-2581.
27. Schelert,J., Drozda,M., Dixit,V., Dillman,A. and Blum,P. (2006) Regulation of mercury resistance in the crenarchaeote sulfolobus solfataricus. J.Bacteriol., 188, 7141-7150.
28. Zillig,W., Kletzin,A., Schleper,C., Hoiz,I., Janekovic,D., Hain,J., Lanzendoerfer,M. and Kristjansson,J.K. (1994) Screening for Sulfolobales, their plasmids and their viruses in Icelandic solfataras. Syst.Appl.Microbiol., 16, 609-628.
29. Reilly,M.S. and Grogan,D.W. (2001) Characterization of intragenic recombination in a hyperthermophilic archaeon via conjugational DNA exchange. J.Bacteriol., 183, 2943-2946.
30. Brock,T.D., Brock,K.M., Belly,R.T. and Weiss,R.L. (1972) sulfolobus. a new genus of sulfur-oxidizing bacteria living at low pH and high temperature. Arch.Mikrobiol., 84, 54-68.
31. Grogan,D.W. and Gunsalus,R.P. (1993) sulfolobus acidocaldarius synthesizes UMP via a standard de novo pathway: results of biochemical-genetic study. J.Bacteriol., 175, 1500-1507.
32. Agron,P.G., Sobecky,P. and Andersen,G.L. (2002) Establishment of uncharacterized plasmids in Escherichia coli by in vitro transposition. FEMS Microbiol.Lett., 217, 249-254.
33. Purschke,W.G. and Schaefer,G. (2001) Independent replication of the plasmids pRN1 and pRN2 in the archaeon sulfolobus islandicus. FEMS Microbiol.Lett., 200, 97-102.
34. Grogan,D.W. (2003) Cytosine methylation by the Sual restriction-modification system: implications for genetic fidelity in a hyperthermophilic archaeon. J.BacterioL, 185, 4657-4661.
35. Kurosawa,N. and Grogan,D.W. (2005) Homologous recombination of exogenous DNA with the sulfolobus acidocaldarius genome: properties and uses. FEMS Microbiol.Lett., 253, 141-149.
36. Berkner,S. and Lipps,G. (2007) Characterization of the Transcriptional Activity of the Cryptic Plasmid pRN1 from Sulfolobus islandicus REN1H1 and Regulation of Its Replication Operon. J.Bacteriol., 189, 1711-1721.
37. Grogan,D.W. (1991) Evidence that beta-Galactosidase of Sulfolobus solfataricus Is Only One of Several Activities of a Thermostable beta-d-Glycosidase. Appl.Environ.Microbiol., 57, 1644-1649.
38. Corbett,K., Fordham-Skelton,A.P., Gatehouse,J.A. and Davis,B.G. (2001) Tailoring the substrate specificity of the beta-glycosidase from the thermophilic archaeon Sulfolobus solfataricus. FEBS Lett., 509, 355-360.
39. Ehresmann,B., Imbault,P. and Weil,J.H. (1973) Spectrophotometric determination of protein concentration in cell extracts containing tRNA's and rRNA's. Anal.Biochem., 54, 454-463.
40. Schelert,J., Dixit,V., Hoang,V., Simbahan,J., Drozda,M. and Blum,P. (2004) Occurrence and characterization of mercury resistance in the hyperthermophilic archaeon Sulfolobus solfataricus by use of gene disruption. J.Bacteriol., 186, 427-437.
41. Keeling,P.J., Klenk,H.P., Singh,R.K., Feeley,O., Schleper,C., Zillig,W., Doolittle,W.F. and Sensen,C.W. (1996) Complete nucleotide sequence of the Sulfolobus islandicus multicopy plasmid pRN1. Plasmid, 35, 141-144.
42. Greve,B., Jensen,S., Brugger,K., Zillig,W. and Garrett,R.A. (2004) Genomic comparison of archaeal conjugative plasmids from sulfolobus. Archaea, 1,231-239.
43. Berkner,S. and Lipps,G. (2007) An Active Nonautonomous Mobile Element in Sulfolobus islandicus REN1 H1. J.Bacteriol., 189, 2145-2149.
44. Matsumi,R., Manabe,K., Fukui,T., Atomi,H. and lmanaka,T. (2007) Disruption of a Sugar Transporter Gene Cluster in a Hyperthermophilic Archaeon Using a Host-Marker System Based on Antibiotic Resistance. J. Bacteriol., 189, 2683-2691.
45. Karlin,S. and Altschul,S.F. (1993) Applications and statistics for multiple high-scoring segments in molecular sequences. PNAS USA, 90, 5873-5877.
46. Altschul,S.F., Madden,T.L., Schaffer,A.A., Zhang,J., Zhang,Z., Miller,W. and Lipman,D.J. (1997) Gapped BLAST and PSI-BLAST: a new generation of protein database search programs. Nucleic Acids Res., 25, 3389-3402. Lipps,G. (2006) Plasmids and viruses of the thermoacidophilic crenarchaeote sulfolobus. Extremophiles, 10, 17-28.

**Table of correspondence**

| SEQ ID NO: | Support |
|---|---|
| 1 | Fig. 14 |
| 2 | Fig. 15 |

## Claims

1. An archaeal plasmid vector system comprising:
I) one or more selectable marker gene(s) for selection in archaea, and
II) a component selected from
a) an archaeal origin of replication,
b) a homology region to an archaeal chromosome,
c) a gene coding for a molecule conferring the ability to integrate the vector into an archaeal chromosome, and
d) gene *orf904;*
wherein the genes of the plasmid vector do not allow an assembly of a functional virus particle.

2. The archaeal plasmid vector system of claim 1 comprising:
I) one or more selectable marker gene(s) for selection in archaea; and
II) a component selected from
a) an archaeal origin of replication,
b) a homology region to an archaeal chromosome, and
c) a gene coding for a molecule conferring the ability to integrate the vector into an archaeal chromosome;
wherein the genes of the plasmid vector do not allow an assembly of a functional virus particle.

3. The plasmid vector system of any one of claims 1 or 2, wherein the plasmid vector system is functional in archaea, preferably in crenarchaea, still preferably in a member of the class Thermoprotei, more preferably in a member of the order Sulfolobales, even more preferably in a member of the family Sulfolobaceae and most preferably in a member of the genus Sulfolobus.

4. The plasmid vector system of any one of claims 1 or 2, wherein the plasmid vector system is functional in archaea, preferably in euryarchaeota, more preferably in thermophilic euryarchaeota and most preferably in Thermococci and/or Thermoplasmata.

5. The plasmid vector system of any one of the foregoing claims, wherein the archaeal origin of replication is selected from archaeal plasmidal, archaeal viral or archaeal chromosomal origins of replication.

6. The plasmid vector system of claim 5, wherein the archaeal plasmidal origin of replication is selected from the archaeal plasmids of the group consisting of pRN1, pRN2, pSSVx, pDL10, pHEN7, pXQ1, pST1, pIT3, pTAU4, pORAI and pTIK4.

7. The plasmid vector system of claim 5, wherein the archaeal plasmidal origin of replication is an origin of replication of the pRN plasmid family.

8. The plasmid vector system of any one of claims 6 or 7, wherein the archaeal plasmidal origin of replication is an origin of replication of the pRN1 plasmid.

9. The plasmid vector system of claim 5, wherein the archaeal chromosomal origin of replication is a chromosomal crenarchaeal origin of replication, preferably an origin of replication of a member of the class Thermoprotei, more preferably of a member of the order Sulfolobales, even more preferably of a member of the family Sulfolobaceae and most preferably of a member of the genus Sulfolobus.

10. The plasmid vector system of claim 9, wherein the member of the genus Sulfolobus is selected from the group consisting of *Sulfolobus acidocaldarius, Sulfolobus islandicus, Sulfolobus metallicus, Sulfolobus neozealandlcus, Sulfolobus shibatae, Sulfolobus solfataricus, Sulfolobus tengchongensis, Sulfolobus thuringiensis, Sulfolobus tokodaii, Sulfolobus yangmingensis* and other cultured *Sulfolobus sp..*

11. The plasmid vector system of claim 5, wherein the archaeal chromosomal origin of replication is a chromosomal euryarchaeal origin of replication, preferably an origin of replication of a thermophilic euryarchaeota, more preferably of Thermococci or Thermoplasmata.

12. The plasmid vector system of any one of claims 1 to 4, wherein the homology region to an archaeal chromosome is a homology region of a crenarchaeal chromosome, preferably a chromosome of a member of the class Thermoprotei, more preferably of the order Sulfolobales, even more preferably of the family Sulfolobaceae and most preferably of the genus Sulfolobus.

13. The plasmid vector system of claim 12, wherein the member of the genus Sulfolobus is selected from the group consisting of *Sulfolobus acidocaldarius, Sulfolobus islandicus, Sulfolobus metallicus, Sulfolobus neozealandicus, Sulfolobus shibatae, Sulfolobus solfataricus, Sulfolobus tengchongensis, Sulfolobus thuringiensis, Sulfolobus tokodaii, Sulfolobus yangmingensis* and other cultured *Sulfolobus sp..*

14. The plasmid vector system of claim 5, wherein the homology region to an archaeal chromosome is a homology region of an euryarchaeal chromosome, preferably a chromosome of a thermophilic euryarchaeota, more preferably of Thermococci or Thermoplasmata.

15. The plasmid vector system of any one of claims 1 to 4 or 12 to 14, wherein the homology region to an archaeal chromosome is 10 to 50,000 bp, preferably 30 to 10,000 bp, more preferably 50 to 5,000 bp and most preferably 100 to 1,000 bp in length.

16. The plasmid vector system of any one of claims 1 to 4, wherein the molecule conferring the ability to integrate the vector into the archaeal chromosome is an integrase or a transposase.

17. The plasmid vector system of claim 16, wherein the integrase is an integrase of the Fuselloviridae.

18. The plasmid vector system of claim 17, wherein the Fuselloviridae is selected from the group consisting of SSV1, SSV2, SSV-K1 and SSV-RH.

19. A plasmid plasmid vector system comprising:
I) gene *orf904,* and
II) one or more selectable marker gene(s);
wherein the vector is non-integrative.

20. The plasmid vector system of any one of the foregoing claims, wherein the selectable marker gene(s) is/are essential gene(s) for a host organism or gene(s) conferring a growth advantage.

21. The plasmid vector system of claim 20, wherein the selectable marker gene(s) is/are selected from the genes coding for orotidine-5'-monophosphate pyrophosphorlyase and/or orotidine-5'-monophosphate decarboxylase, or HMG-CoA reductase.

22. The plasmid vector system of any one of the foregoing claims, wherein the vector is a cloning vector.

23. The plasmid vector system of any one of the foregoing claims, wherein the vector is an expression vector.

24. The plasmid vector system of any one of the foregoing claims, wherein the vector is a shuttle vector.

25. The plasmid vector system of any one of the foregoing claims, wherein the vector further comprises a gene of interest to be expressed.

26. The plasmid vector system of claim 25, wherein the vector comprises a gene of interest to be expressed in archaea.

27. The plasmid vector system of claim 26, wherein the vector comprises a gene of interest to be expressed in crenarchaea, preferably a member of the class Thermoprotei, more preferably a member of the order Sulfolobales, even more preferably a member of the family Sulfolobaceae and most preferably a member of the genus Sulfolobus.

28. The plasmid vector system of claim 27, wherein the member of the genus Sulfolobus is selected from the group consisting of *Sulfolobus acidocaldarius, Sulfolobus islandicus, Sulfolobus metallicus, Sulfolobus neozealandicus, Sulfolobus shibatae, Sulfolobus solfataricus, Sulfolobus tengchongensis, Sulfolobus thuringiensis, Sulfolobus tokodaii, Sulfolobus yangmingensis* and other cultured *Sulfolobus sp..*

29. The plasmid vector system of claim 26, wherein the vector comprises a gene of interest to be expressed in euryarchaeota, preferably in thermophilic euryarchaeota, more preferably in Thermococci and/or Thermoplasmata.

30. The plasmid vector system of any one of the foregoing claims, wherein the vector further comprises an *E. coli* origin of replication.

31. The plasmid vector system of any one of the foregoing claims, wherein the vector further comprises one or more selectable marker gene(s) for selection in *E. coli.*

32. The plasmid vector system of claim 31, wherein the selectable marker gene(s) for selection in *E. coli* is/are gene(s) conferring antibiotic resistance.

33. The plasmid vector system of any one of the foregoing claims, wherein the vector comprises an additional expression cassette for a reporter molecule.

34. The plasmid vector system of claim 33, wherein the reporter molecule is selected from the group consisting of β-galactosidase, luciferase, green fluorescent protein and variants thereof.

35. The plasmid vector system of any one of claims 1 to 4, wherein the vector comprises one or more selectable marker gene(s) for selection in archaea selected from essential gene(s) for a host organism and gene(s) conferring a growth advantage, and an archaeal origin of replication, wherein the archaeal origin of replication is an archaeal plasmidal origin of replication; wherein the genes of the plasmid vector do not allow an assembly of a functional virus particle.

36. The plasmid vector system of any one of claims 1 to 4 or 35, wherein the vector comprises one or more selectable marker gene(s) for selection in archaea selected from the genes coding for orotidine-5'-monophosphate pyrophosphorlyase and/or orotidine-5'-monophosphate decarboxylase, or HMG-CoA reductase, and an archaeal origin of replication, wherein the archaeal origin of replication is an origin of replication of the pRN1 plasmid; wherein the genes of the plasmid vector do not allow an assembly of a functional virus particle.

37. The plasmid vector system of any one of claims 1 to 4, wherein the vector comprises one or more selectable marker gene(s) for selection in archaea selected from essential gene(s) for a host organism and gene(s) conferring a growth advantage, and a homology region to an archaeal chromosome; wherein the genes of the plasmid vector do not allow an assembly of a functional virus particle.

38. The plasmid vector system of any one of claims 1 to 4 or 37, wherein the vector comprises one or more selectable marker gene(s) for selection in archaea selected from the genes coding for orotidine-5'-monophosphate pyrophosphorlyase and/or orotidine-5'-monophosphate decarboxylase, or HMG-CoA reductase, and a homology region to an archaeal chromosome, wherein the homology region to an archaeal chromosome is 10 to 50,000 bp, preferably 30 to 10,000 bp, more preferably 50 to 5,000 bp and most preferably 100 to 1,000 bp in length; wherein the genes of the plasmid vector do not allow an assembly of a functional virus particle.

39. The plasmid vector system of any one of claims 1 to 4 or 37, wherein the vector comprises one or more selectable marker gene(s) for selection in archaea selected from the genes coding for orotidine-5'-monophosphate pyrophosphorlyase and/or orotidine-5'-monophosphate decarboxylase, or HMG-CoA reductase, and a homology region to an archaeal chromosome, wherein the homology region to an archaeal chromosome is a homology region of a crenarchaeal chromosome, preferably a chromosome of a member of the class Thermoprotei, more preferably of the order Sulfolobales, even more preferably of the family Sulfolobaceae and most preferably of the genus Sulfolobus; wherein the genes of the plasmid vector do not allow an assembly of a functional virus particle.

40. The plasmid vector system of any one of claims 35 to 39, wherein the vector further comprises an *E. coli* origin of replication and one or more selectable marker gene(s) for selection in *E. coli.*

41. A host cell transformed with the plasmid vector system of any one of the foregoing claims.

42. The host cell of claim 41, wherein the host cell is an archaeal cell.

43. The host cell of claim 42, wherein the archaeal cell is a crenarchaeal cell, preferably a cell of a member of the class Thermoprotei, more preferably of a member of the order Sulfolobales, even more preferably of a member of the family Sulfolobaceae and most preferably of a member of the genus Sulfolobus.

44. The host cell of claim 43, wherein the member of the genus Sulfolobus is selected from the group consisting of *Sulfolobus acidocaldarius, Sulfolobus islandicus, Sulfolobus metallicus, Sulfolobus neozealandicus, Sulfolobus shibatae, Sulfolobus solfataricus, Sulfolobus tengchongensis, Sulfolobus thuringiensis, Sulfolobus tokodaii, Sulfolobus yangmingensis* and other cultured *Sulfolobus sp..*

45. The host cell of claim 42, wherein the archaeal cell is an euryarchaeal cell, preferably a cell of a thermophilic euryarchaeota, more preferably of Thermococci and/or Thermoplasmata.

46. The host cell of claim 41, wherein the host cell is *E coli.*

47. The host cell of any one of claims 41 to 46, wherein the host cell produces a protein of interest.

48. A method of producing a protein of interest comprising culturing the host cell of any one of claims 41 to 46 under suitable conditions and isolating the protein from the cell or cell culture supernatant.

49. Use of the plasmid vector system of any one of claims 1 to 40 or the host cell of any one of claims 41 to 46 for producing a protein of interest.

50. A kit comprising the plasmid vector system of any one of claims 1 to 40 or the host cell of any one of claims 41 to 46 in one or more container(s).
